# EUROPEAN PATENT SPECIFICATION

(11) **EP 0 842 289 B1**
(45) Date of publication and mention of the grant of the patent: **20.10.2004**
(21) Application number: 95944705.3
(22) Date of filing: 26.07.1995
(51) Int. Cl.: C12N 15/90, C12N 15/85

(54) **HOMOLOGOUS RECOMBINATION IN MISMATCH REPAIR INACTIVATED EUKARYOTIC CELLS**
HOMOLOGE REKOMBINATION IN EUKARYOTISCHEN ZELLEN MIT INAKTIVIERTEM FEHLPAARUNGSREPARATUR-SYSTEM
RECOMBINAISON HOMOLOGUE DANS DES CELLULES EUCARYOTES A SYSTEME DE REPARATION DES MESAPPARIEMENTS INACTIVE

(43) Date of publication of application: 20.05.1998
(73) Proprietor: Mixis France S.A., 75014 Paris (FR)
(72) Inventor: TE RIELE, Henricus, Petrus, Joseph, NL-1053 XB Amsterdam (NL); DE WIND, Niels, NL-1011 TT Amsterdam (NL); DEKKER-VLAAR, Helena, Maria, Johanna, NL-1713 VC Obdam (NL)
(74) Representative: Schrell, Andreas, Dr.
(86) International application number: PCT/EP1995/002980
(87) International publication number: WO 1997/005268

(56) References cited:
- WO-A-90/07576
- WO-A-93/01292
- GENETICS, vol. 139, May 1995, pages 1175-1188, XP000572769 E. SELVA ET AL: "Mismatch correction acts as a barrier to homologous recombination in Saccharomyces cerevisiae"
- NATURE, vol. 342, 23 November 1989, pages 396-400, XP002006049 C. RAYSSIGUIER: "The barrier to recombination between Escherichia coli and Salmonella typhimurium is disrupted in mismatch-repair mutants" cited in the application
- PROCEEDINGS OF THE NATIONAL ACADEMY OF SCIENCES OF USA, vol. 89, June 1992, WASHINGTON US, pages 5128-5132, XP002006050 H. TE RIELE ET AL: "Highly efficient gene targeting in embryonic stem cells through homologous recombination with isogenic DNA constructs" cited in the application
- NUCLEIC ACIDS RESEARCH, vol. 22, no. 25, 1994, OXFORD GB, pages 5723-5728, XP002006051 I. VARLET ET AL: "Cloning and expression of the Xenopus and mouse Msh2 DNA mismatch repair genes" cited in the application

## Description

### BACKGROUND OF THE INVENTION

The introduction of specific modifications in the prokaryotic and eukaryotic genome is a powerful tool in studying gene function both at the level of individual cells and in the context of a complete organism. in addition, modification of specific genes may result in the generation of industrially and medically important organisms, whereas correction of defective alleles in eukaryotic cells may provide a substantial step forward in the development of somatic gene-therapy protocols.

The method of genetic modification relies on the ability of virtually every cell type to exchange DNA sequences with a high degree of nucleotide sequence similarity by a process which is called homologous recombination (1). Briefly, the method of genetic modification involves the generation of a so-called targeting construct, a DNA sequence that is largely identical to the specific chromosomal locus to be modified, but differing from this locus by specific modifications. These modifications can be as small as the deletion, insertion or substitution of a single base-pair, or be as large as the deletion or insertion of ten's of kilobasepairs. On entry of the targeting construct into the cell, exchange of sequences flanking the modification with their chromosomal counterparts, will result in the introduction of the modification into the recipient chromosome.

The efficiency of homologous recombination in both prokaryotes and eukaryotes strongly depends on complete sequence identity of exchanging DNA strands (2-5). Thus. sequence dissimilarities as small as 0.5% can already strongly impede homologous recombination. In several bacterial species, *Escherichia coli*, *Salmonella typhimurium* and *Streptococcus pneumoniae* as well as in the yeast *Saccharomyces cerevisiae* it has been unequivocally shown that the DNA mismatch repair system is responsible for suppressing recombination between homologous but nonidentical DNA sequences (6-8).

We have now demonstrated that in mammalian cells deficient for the DNA mismatch repair gene *Msh2,* homologous recombination has lost the requirement for complete sequence identity between exchanging DNA sequences. This finding provides a new method for modifying the eukaryotic genome using DNA targeting constructs which substantially differ from the target locus in the region where recombination takes place by genetically and/or functionally inactivating the cell's mismatch correction system.

The method obviates the requirement for DNA targeting constructs that are largely identical to the target locus, thus allowing the efficient genetic modification of both somatic cells and the germ line of outbred organisms. This may provide a route to somatic gene therapy and the modification of eukaryotic species of which inbred strains are not easily available. Also, genomic sequences can be replaced by small oligonucleotides carrying one or more basepair alterations or by large chromosomal sequences derived from other species. The method can also be used to generate large deletions by intra- or extrachromosomal homologous recombination between repeated but diverged sequences.

### Genetic modification of mice

The introduction of specific genetic alterations into the germ line of mice was made possible by a combination of new techniques in molecular biology and embryology that have become available during the last ten years (9-11). The method entails the introduction of a planned genetic modification in embryonic stem (ES) cells by homologous recombination. ES cells are originally derived from the inner cell mass of 3.5 day old pre-implantation embryos and can be maintained by *in vitro* culture as immortalized cell lines, retaining the undifferentiated state under appropriate culture conditions. At present, a number of ES cell lines are available. ES cetts injected into the blastocoel of 3.5 day blastocyst-stage embryos can efficiently compete with the inner-cell-mass cells of the recipient blastocyst in embryonic development thus generating a chimeric mouse consisting of cells derived from the recipient blastocyst and the injected ES cells. Should the *in vitro* modified ES cells contribute to the germ line of chimeric animals, the ES cell genome can be transmitted to the next generation giving animals carrying the introduced modification in all their cells.
Intercrossing of such animals reveals the phenotypic consequences of homozygosity of the modified gene.

The most commonly introduced genetic modification in ES cells is gene disruption leading to gene inactivation. This allows to study gene function by analyzing the consequences of the absence of a particular gene in the context of a complete organism. Also, many hereditary human diseases result from hemizygosity of specific genes [e.g. cancer predisposition syndromes like hereditary nonpolyposis colorectal cancer (12). Li Fraumeni syndrome (13), retinoblastoma (14)]. The generation of mouse models for such diseases by disruption of the mouse homologs of the genes involved provides an invaluable tool for studying hereditary diseases in an experimental setting.

### Gene disruption in ES cells

The inactivation of a specific gene in ES cells via homologous recombination starts with the preparation of a DNA targeting construct (15). Two types of targeting constructs can be used. In a replacement-type vector, a drug-resistance marker gene (conferring to the cell resistance to drugs like G418, Hygromycin B, Puromycin, Histidinol) disrupts a sequence which is homologous to a sequence in or around the target gene in the recipient genome; in an insertion-type vector the marker gene flanks the homologous sequence. The targeting construct is introduced into ES cells by electroporation (or alternatively by Ca-Phosphate precipitation, lyposome-mediated DNA transfer or micro-injection) and cells are selected for stable integration of the targeting construct into the recipient genome by growth in medium containing the appropiate drug. Drug-resistant colonies are the result of either one of two events: integration of the targeting construct at a random site in the genome or integration of the marker gene in the target locus via homologous recombination between the flanking sequences and their chromosomal counterparts. The replacement-type vector integrates by homologous recombination on both sides of the marker gene; the insertion-type vector integrates by a single homologous recombination event leading to duplication of the region of homology. Thus, the marker gene serves two purposes: it allows selection of cells that have taken up the targeting construct and, on integration via homologous recombination, it will disrupt the target gene thereby modifying its function.
Distinguishing ES cell clones resulting from homologous recombination from those resulting from random integration, requires the DNA of individual clones to be analysed by Southern hybridisation or the polymerase chain reaction.

Unfortunately, in many targeting experiments, random integration was often found to be far more efficient than homologous recombination and also large variations in targeting efficiency were observed for different genes (16). In this respect, mammalian cells differ from bacteria and lower eukaryotes like yeast (17), *Leishmania major* (18) or *Trypanosoma brucei* (19), where integration of exogenous DNA into the recipient genome exclusively or predominantly occurs via homologous recombination. To date, three factors clearly affecting the recovery of homologous recombinants in mammalian cells have been identified. First, the frequency of homologous recombination increases substantially with the total length of the homologous sequences up to 14 kilobase-pairs (20). Second, the expression level of the marker gene at the target locus affects the frequency of recovery of homologous recombinants: low expression may lead to loss of homologous recombinants, whereas high expression may allow selection of homologous recombinants at an elevated drug concentration (21). Third, sequence dissimilarities between the targeting construct and the chromosomal target locus strongly suppress the efficiency of homologous recombination (5).

### High efficiency targeting with isogenic DNA constructs

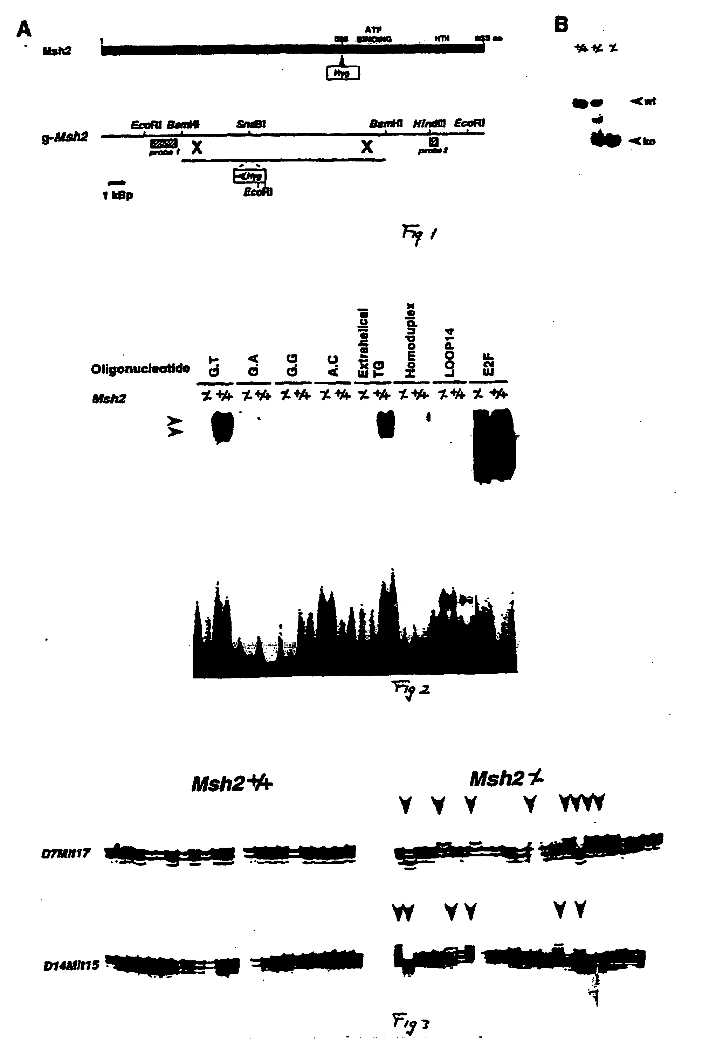

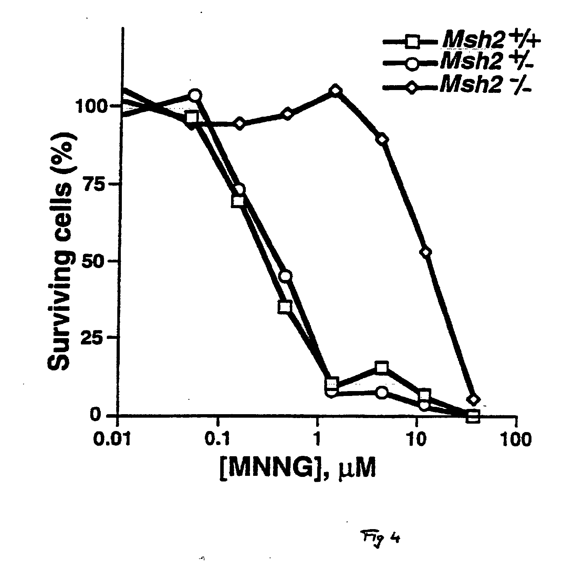

The'suppression of homologous recombination in ES cells by small sequence dissimilarities became clear by a gene targeting experiment aimed at disrupting the *Retinoblastoma* gene with a neomycin resistance marker gene. Two targeting constructs were prepared carrying the neomycin resistance marker embedded in 10.5 kb of *Rb* sequence. In one construct the *Rb* sequence was derived from mouse strain 129, and was therefore identical to the corresponding chromosomal locus in the ES cells, which were also derived from mouse strain 129. In the other construct, the *Rb* sequence was derived from mouse strain BALB/c. The two constructs, designated 129Rb-neo and B/cRb-neo are described in the accompanying publication ***Proc*. *Natl*. *Acad*. *Sci. USA,* Vol. 89, pp. 5128-5132** on pages 5128 and 5129, Fig. 1. The two constructs contained corresponding *Rb* sequences and were therefore largely similar. However, they differed approximately 0.6% at the nucleotide level (which corresponds to the level of sequence polymorphism found in the human population). Thus, in a stretch of 1687 basepairs that was sequenced, the BALB/c sequence differed from the 129 sequence by 9 basepair substitutions, three small deletions (of 1,4 and 6 nucleotides) and two polymorphic CA-repeats (see ***Proc*. *Natl. Acad*. *Sci*. *USA,* Vol. 89, pp. 5128-5132**, page 5130, Fig. 4). On introduction of these constructs in 129-derived ES cells, homologous recombination at *Rb* with the 129-derived construct was 50-fold more efficient than with the nonisogenic BALB/c-derived construct. To provide additional evidence that the suppression of recombination was solely dependent on the polymorphisms between the endogenous locus and the targeting DNA, the inverse experiment was performed, i.e. targeting of a BALB/c-derived ES cell line with the 129- and BALB/c-derived constructs. This experiment yielded the inverse result, i.e. a higher targeting efficiency with the BALB/c-derived construct than with the nonisogenic 129-derived construct.
With a somewhat different targeting construct, consisting of a hygromycin resistance gene embedded in 17 kb of isogenic *Rb* DNA, we observed that 80% of all Hygromycin B-resistant colonies resulted from homologous recombination (5). This demonstrates that, in the presence of perfect homology, also in mammalian cells homologous recombination rather than random integration can be the predominant event.

Although clearly not all problems of gene targeting have been solved, many genes have now been successfully targeted by the use of isogenic DNA targeting constructs. However, genetic modification of cells derved from an outbred organism can become a difficult endeavour as isogenic targeting constructs are not easily available. In this case, efficient gene targeting would require the targeting construct to be prepared from DNA derived from the target cell. Especially in the context of gene therapy, this would raise a tremendous practical obstacle to correction of a defective gene. Also, base sequence divergence imposes a major barrier to exchanging a large chromosomal region of one species by the syntenic region of another species. The present invention provides a way to overcome these problems.

### The introduction of subtle mutations

Although protocols for disruption of genes in inbred ES cell lines (and in somatic cell lines of which isogenic DNA targeting constructs can be prepared) are rather well developed, the introduction of more subtle mutations is not straightforward. Current protocols are variations on a two-step procedure in which first a marker gene is introduced into the target gene followed by replacement of the marker gene by the desired subtle mutation (5). This procedure requires the marker gene to be selectable both for its presence (first step) and its absence (replacement by the subtle mutation).

Useful marker genes are the *Hprt* minigene to be used in *Hprt*-deficient ES cells (positive selection in HAT medium; negative selection by 6 thioguanine) and a combination of the neomycin resistance gene (positive selection by G418) and the Herpes Simplex Virus thymidine kinase gene (negative selection by Gancyclovir). In an altemative procedure, the subtle mutation and the marker gene are present on the same targeting construct and concomittantly introduced into the genome by homologous recombination. If an insertion-type vector was used, the marker gene can be removed by intrachromosomal recombination between the duplicated sequences that were generated during the first integration event (23). In case of a replacement-type vector, the marker gene can be removed if it was flanked by two site-specific-recombination sites (e.g. *IoxP* sites). Recombination between these sites on introduction into the cell of the *loxP*-specific recombinase *Cre* will remove the marker gene from the genome (24).

Although either of the above mentioned procedures has allowed the subtle modification of a number of genes in ES cells, they are highly demanding as to the generation of appropiate DNA targeting constructs and the culturing of ES cells under various selective conditions.
Therefore, an attractive alternative to these procedures might be the use of small single- or double-stranded oligonucleotides (up to 100 bases or basepairs), which are identical to the target locus except for one or several basepair alterations. However, our finding that base sequence dissimilarities as small as 0.6% strongly suppress homologous recombination, may impose a major impediment to using such oligonucleotides for the introduction of subtle genetic modifications. The present invention provides a way to overcome this problem and may allow the subtle modification of cell lines and cells derived from living organisms and temporarily cultured *in vitro*.

### GENETIC MODIFICATION OF EUKARYOTIC CELLS USING DNA CONSTRUCTS WITH SUBSTANTIAL BASE SEQUENCE DIFFERENCES WITH RESPECT TO THE TARGET LOCUS.

As described above, base sequence dissimilarities as modest as 0.6% impose a strong barrier to efficient homologous recombination in mouse embryonic stem cells (5). Suppression of homologous recombination by small base sequence divergencies was earlier observed in bacteria (*E*. *coli, S. typhimurium* and *S*. *pneumoniae*), yeast and mouse fibroblasts (2-4). The role of the DNA mismatch correction system in suppressing recombination between homologous but diverged sequences, was most dramatically demonstrated by Radman and coworkers in studying bacterial conjugation between the related but diverged species *Escherichia coli* and *Salmonella typhimurium* (6). This process relies on entry of chromosomal fragments of one species into the other and recombination of these fragments with the chomosome of the recipient bacterium. The sequence divergence between the two species is estimated to be 20-30% and therefore the recovery of exconjugants from an interspecies cross is about 2x10⁵ fold lower than from an intraspecies cross. However, the recovery of exconjugants from the interspecies cross increased 3x10³ fold if the recipient bacteria carried an inactivating mutation in either the *mutS* or *mutL* gene, both of which being essential for DNA mismatch correction.

The central protein in DNA mismatch correction in *E coli* is encoded by the *mutS* gene (25). It recognizes and binds to base mispairs and small loops of up to four unpaired nucleotides. After binding to heteroduplex DNA, the MutS-DNA complex is bound by the *mutL* gene product which leads to excision of a tract of single-stranded DNA of up to several kilobases that contains the mispaired nucleotide(s). In this process also the *mutU* gene product plays a role, whereas the *mutH* gene product ensures removal of newly synthesized strands rather than parental strands. The repair process is completed by resynthesis of the excised strand and ligation of the remaining nick. Mismatch repair in *E. coli* is responsible for maintaining genome stability in at least two ways: i) by recognizing and repairing mis- and unpaired nucleotides that occur, respectively, by misincorporation and slippage during DNA replication; ii) by recognizing mismatches occurring in heteroduplexes formed at initial stages of recombination between homologous but not-identical sequences. This may either lead to blocking elongation of heteroduplex formation or dissociation of the heteroduplex thus aborting the recombination reaction. Consequently, *E. coli* strains defective lor either *mutS* or *mutL*, have a pleiotropic phenotype: an increased mutation rate, including destabilization of simple-sequence repeats and an increased rate of recombination between homologous but diverged DNA sequences. The latter phenotype is clearly manifested by the efficient recovery of recombinant bacteria resulting from conjugational crosses between the related but diverged species *Escherichia coli* and *Salmonella typhimurium* wherein the recipient bacterium was deficient for *mutS* or *mutL* (6). Also, the frequency of chromosomal rearrangements by ectopic recombination between diverged sequences is substantially elevated in mismatch repair deficient bacteria (26).

In many respects, the biochemistry of mismatch repair systems in eukaryotes resembles that of the *E. coli mutS,L* system. Homologs of both genes have been identified in yeast and mammalian cells. Based on mismatch binding *in vitro*, and on the mutator and recombinator phenotypes of *Saccharomyces cerevisiae* mutants, the protein encoded by the yeast *MSH2* gene seems to be the functional homolog of MutS (27-29). A homolog of the yeast *MSH2* gene was identified in mammalian cells by analysis of a G-T-mismatch-binding activity, positional cloning and PCR amplification of mouse DNA using degenerate primers (30). Similarly, homologs of the *E. coli mutL* gene were identified in yeast and mammalian cells.

Interestingly, inherited mutations in human *mutS* and *mutL* fiomologs were recently found to be rotated to the cancer predisposition syndrome HNPCC (hereditary nonpolyposis colorectal cancer), which is characterized by development of tumors of the proximal colon at early age. In these tumors, mismatch repair is lost, as manifested by destabilization of simple sequence repeats, the replication error-positive (RER⁺) phenotype (12).

WO 90/07576 relates to an in vivo recombination process for partially homologous DNA-sequences in organisms, whose enzymatic system for repairing mismatches is deficient.

Selva et al. (8) provides some findings on the role of Msh2 and Msh3 on recombination fidelity in *Saccharomyces cerevisiae*.

However, there is no indication in the prior art on how to reliably modify the genome of a mammalian cell by homologous recombination between a specific target locus in the genome and a homologous but diverged targeting DNA sequence introduced into the cell.

The present invention solves this problem by providing a method for modifying the genome of a non-human mammalian cell deficient for the DNA mismatch repair gene Msh2 in vitro by homologous recombination between a specific target locus in the genome and a homologous but diverged targeting DNA sequence introduced into the cell deficient for the DNA mismatch repair gene Msh2 and said targeting sequence has up to 30% base sequence differences with respect to the target locus in the region where recombination can take place or said targeting sequence differs from the target locus to such a level that homologous recombination is normally strongly impeded.

The present invention, in a preferred embodiment (embodiment a), relates to the above-identified method for modifying the genome of a non-human mammalian cell, wherein the cell's mismatch repair system gene Msh2 is inactivated by disruption of both copies of the Msh2 gene.

The present invention, in a further preferred embodiment (embodiment b), relates to the above-identified method for modifying the genome of a non-human mammalian cell, wherein the cell's mismatch repair system gene Msh2 is inactivated by introduction of antisense RNA constructs, driven by an appropriate promoter, thus functionally inactivating the Msh2 gene.

The present invention, in a further preferred embodiment (embodiment c), relates to the above-identified method for modifying the genome of a non-human mammalian cell, wherein the cell's mismatch repair system gene Msh2 is inactivated by introduction of modified antisense oligodeoxynucleotides, thus functionally inactivating the Msh2 gene.

The present invention, in a further preferred embodiment (embodiment d), relates to the above-identified method for modifying the genome of a non-human mammalian cell, wherein the cell's mismatch repair system gene Msh2 is inactivated by expression of a dominant negatively acting version of a gene involved in DNA mismatch repair.

The present invention, in a further preferred embodiment (embodiment e), relates to the above-identified method for modifying the genome of a non-human mammalian cell, wherein the cell's mismatch repair system gene Msh2 is inactivated transitorily by saturation of the mismatch repair system gene Msh2 by the introduction into the cell of DNA molecules carrying one or more mis- or unpaired bases.

The present invention, in a preferred embodiment, relates to the above-identified methods for modifying the genome of a non-human mammalian cell, wherein the target cell is a non-human embryonic stem cell or a related cell type derived from any mammalian species, to be used to generate chimeric animals and chimeric animals that can transmit the mutated genome through the germ line.

The present invention, in a further preferred embodiment (embodiment 1), relates to the above-identified method for modifying the genome of a non-human mammalian cell, wherein the target cell is a cell line derived from any mammalian species and cultured in vitro.

The present invention, in a further preferred embodiment (embodiment 2), relates to the above-identified method for modifying the genome of a non-human mammalian cell, wherein the target cell is derived from any organ of any mammalian species.

The present invention, in a further preferred embodiment (embodiment 3), relates to the above-identified method for modifying the genome of a non-human mammalian cell, wherein the target cell is derived from outbred organisms implicating that they contain nonidentical copies of homologous chromosomes.

The present invention, in a preferred embodiment (embodiment 4), relates to the above-identified method for modifying the genome of a non-human mammalian cell, wherein the targeting DNA at the region where homologous recombination can take place is derived from the same species as the target cell, in particular a method for modifying the genome of a non-human mammalian cell, wherein the targeting DNA is modified in vitro at specific sites thus deliberately introducing sequence divergence (embodiment 5).

The present invention, in a preferred embodiment (embodiment 6), relates to the above-identified method for modifying the genome of a non-human mammalian cell, wherein the targeting DNA at the region where homologous recombination can take place is derived from the same species as the target cell but is nonisogenic with the DNA of the target cell, the two sequence differing up to 5% at the nucleotide level.

The present invention, in a further preferred embodiment (embodiment 7), relates to the above-identified method for modifying the genome of a non-human mammalian cell, wherein the targeting DNA at the region where homologous recombination can take place is derived from another species as the target cell, the targeting sequence and the target locus comprising up to 30% sequence divergence in the region where homologous recombination can take place, in particular the targeting DNA is a chromosomal DNA fragment carried on a YAC or cosmid vector (embodiment 8).

The present invention, in a preferred embodiment (embodiment 9), relates to the above-identified method for modifying the genome of a non-human mammalian cell, wherein the targeting DNA is an double-or single-stranded oligonucleotide consisting of 10-100 bases or basepairs of which one or several bases or basepairs differ from the target locus.

The present invention relates also to a method to test inactivation of the cellular mammalian mismatch repair system gene Msh2 using the above-identified methods according to any one of embodiments a to e and involving comparison of the targeting efficiency of isogenic versus non-isogenic DNA targeting constructs.

The present invention also relates to a method to test inactivation of the cellular mismatch repair system gene Msh2 using the above identified methods according to embodiments a to e and involving intrachromosomal recombination between homologous, but diverged DNA sequences.

The present invention also relates to a method to test homologous recombination using the method according to any one of embodiments 4 to 9 and using a non-human embryonic stem cell line or any other cell-line in which the mismatch repair system is inactivated by disruption of both copies of the Msh2 gene.

The present invention also relates to a method according to any one of embodiments 1, 2 or 3, providing cells or cell-lines for mismatch repair deficient mice carrying a disruption in both copies of the Msh2 gene.

In order to investigate the role of the mammalian *Msh2* gene in DNA mismatch repair and to address the role of mismatch repair in maintaining genome stability, we generated an ES cell line carrying a disruption in both copies of the mouse *MSH2* gene. This line is disignated dMsh2-9. Its construction is herein described.

As demonstrated the phenotypic consequences of *Msh2* deficiency in mouse embryonic stem cells provide clear evidence for an essential role of MSH2 in mammalian DNA mismatch repair. First, Msh2-deficient ES cells lack binding activity to a double stranded 38-mer oligonucleotide carrying a G-T mismatch or an unpaired TG dinucleotide (legend to Fig.2) (Fig.2). Second; *Msh2*-deficient ES cells have a mutator phenotype as evidenced by an at least 150-fold increase in the number of cells resistant to 6-thioguanine, indicating mutational inactivation of the X-linked *Hprt* gene. Moreover, microsatellite length instability was observed in subclones derived from the *Msh2*-deficient ES cell line, but not in subclones derived from wild-type ES cells (legend to Fig.3) (Fig.3). Third, *Msh2-*deficient ES cells resisted a 20-fold higher concentration of the methylating agent N-Methyl-N'-Nitro-N-Nitrosoguanidine than wild-type ES cells (legend to Fig.4) (Fig. 4).

Thus, disruption of *Msh2* leads to inactivation of mammalian DNA mismatch repair.

By using a gene targeting assay, we demonstrated that it is the mammalian DNA mismatch repair system which is responsible for suppressing homologous recombination between sequences differing as little as 0.6% at the nucleotide level. As described above, homologous recombination at the *Rb* lccus with an isogenic DNA targeting construct was 50-fold more efficient than with a similar, but nonisogenic construct containing on the average 0.6% sequence divergence with respect to the target locus (5). However, in *Msh2*-deficient ES cells, homologous recombination at *Rb* with the nonisogenic targeting construct was as efficient as with the isogenic construct.

The present experiment demonstrates that it is the mammalian DNA mismatch repair system which is involved in preventing recombination between homologous but diverged DNA sequences.

This finding provides a method for modifying the mammalian genome but also the genome of any other eukaryotic organism via homologous recombination using DNA targeting sequences that substantially differ from the target locus with respect to the nucleotide sequence, by genetic or furictional inactivation of the cell's mismatch repair system. GENETIC MODIFICATION OF EUKARYOTIC CELLS USING DNA CONSTRUCTS WITH SUBSTANTIAL BASE SEQUENCE DIFFERENCES WITH RESPECT TO THE TARGET LOCUS.
1. Kucherlapati, R., and Smith, G.R., eds. (1988) Genetic Recombination. American Society for Microbiology, Washington.
2. Shen, P., and Huang, H.V. (1986) Genetics *112*,441-457.
3. Nassif. N., and Engels, W. (1993) Proc. Natl. Acad. Sci. USA *90*, 1262-1266.
4. Waldman, A.S., and Liskay, R.M. (1988) Mol. Cell. Biol. *8*, 5350-5357.
5. te Riele, H., Robanus Maandag, E., and Berns, A. (1992) Proc. Natl. Acad. Sci. USA *89*, 5128-5132.
6. Rayssiguier, C., Thaler, D.S., and Radman, M. (1989) Nature *342*, 396-401.
7. Claverys, J.P., and Lacks, S.A. (1986) Microbiol. Rev. *50*, 133-165.
8. Selva, E., New, L., Crouse, G., and Lahue, R.S. (1995) Genetics *139*, 1175-1188.
9. Capecchi, M. (1989) Science *244*, 1288-1292.
10. Frohman, M.A., and Martin, G.R. (1989) Cell *56*, 145-147.
11. Hogan, B., Beddington, R., Constantini, F., and Lacy; E. (1994) Manipulating the mouse embryo, 2nd edition, Cold Spring harbor Laboratory Press, Plainview, New York.
12. Modrich, P. (1994) Science *266*, 1959-1960.
13. Malkin et al. (1990) Science *250*, 1233-1238.
14. Hansen, M.F., and Cavenee, W.K. (1988) Trends Genet. *4*, 125-128.
15. Thomas, K.R., and Capecchi, M.R. (1987) Cell *51*, 503-512.
16. Camerini-Otero, R.D., and Kucherlapati, R. (1990) The New Biologist *2*, 337-341.
17. Sulston, J.E., and Horvitz, H.R. (1977) Devl Biol. *56*, 110-156.
18. Cruz, A., and Beverly, S.M. (1990) Nature *348,* 171-173.
19. Ten Asbroek, A., Ouellette, M., and Borst, P. (1990) Nature *348*, 174-175.
20. Deng, C., and Capecchi, M. (1992) Mol. Cel. Biol. *12*, 3365-3371.
21. Hanson, K.D., and Sedivy, J.M. (1995) Mol. Cel. Biol. *15*, 45-51.
22. Askew, G., Doetchman, T, and Lingrel, J. (1993) Mol. Cell. Biol. *13*, 4115-4124.
23. Hasty, P.et al. (1993) Nature *350*, 243-246.
24. Kilby, N., Snaith, M., and Murray, J. (1993) Trends Genet. *9*, 413-421.
25. Modrich, P. (1991) Annu. Rev. Genet. *25*, 229-253.
26. Petit, M.-A. et al. (1991) Genetics *129*, 327-332.
27. Reenan, R.A.G., and Kolodner, R. (1992) Genetics *132*, 975-985.
28. Miret, J.J., Milla, M.G., and Lahue, R.S. (1993) J. Biol. Chem. *268*, 3507-3513.
29. Alani, E., Chi, N.-W., and Kolodner, R. (1995) Genes Dev. *9*, 234-247.
30. Varlet, I. et al. (1994) Nucl. Acids Res. *22*, 5723-5728.

To investigate the role of the presumed DNA mismatch repair (MMR) gene *Msh2* in genome stability and tumorigenesis, we have generated cells and mice that are deficient for the gene. *Msh2*-deficient cells have lost mismatch binding, and have acquired microsatellite instability, a mutator phenotype and tolerance to methylating agents. Moreover, in these cells, homologous recombination has lost dependence on complete identity between interacting DNA sequences, suggesting that *Msh2* is involved in safeguarding the genome from promiscuous recombination. *Msh2*-deficient mice display no major abnormalities, but a significant fraction develops lymphomas at early age. Thus, *Msh2* is involved in MMR, controlling several aspects of genome stability; loss of MMR-controlled genome stability predisposes to cancer.

Cancer is the endpoint of an evolutionary process in which normal cells acquire full malignancy by cumulative genetic or epi-genetic alterations, each conferring proliferative, invasive or metastatic potential to the cell. In colorectal cancer, at least six independent transforming mutations in oncogenes or tumor suppressor genes are required for a cell to develop into a fully malignant tumor (reviewed by Vogelstein and Kinzler. 1993). This evolutionary process might be dramatically accelerated by the early loss of systems that safeguard genome stability, resulting in an increased rate of mutagenesis and chromosomal rearrangements (Hartwell, 1992 and Loeb, 1994). In support of this hypothesis, a number of hereditary cancer predisposition syndromes are attributed to defects in DNA repair. Two of these are Hereditary Nonpolyposis Colorectal Cancer (HNPCC) and Muir-Torre syndrome. which are characterized by the development of tumors of the proximal colon at early age. Also cancers of other parts of the gastrointestinal tract and of the genitourinary tract are often found (Lynch et al., 1993). Muir-Torre patients are, in addition, prone to cancers of the sebaceous glands and the skin (Cohen et al., 1991). Individuals affected by one of these syndromes have most likely inherited a mutant allele of either one of four mismatch-repair (MMR)-related genes called *MSH2* , *MLH1*, *PMS1* or *PMS2* (Leach et al., 1993; Bronner et al., 1994; Kolodner et al., 1994; Liu et al., 1994, Nicolaides et al., 1994; Papadopoulos et al., 1994; Kolodner et al., 1995; reviewed by Jiricny, 1994 and by Modrich, 1994). In tumors of these patients, MMR is lost, presumably through inactivation or loss of the wild-type allele (Leach et al., 1993; Nicolaides et al., 1994; Papadopoulos et al., 1994; Hemminki et al. 1994). The mechanisms of loss of MMR, the cause of the apparent tissue specificity of tumorigenesis and the role of loss of MMR in the evolution of a normal cell to fully malignant tumors are still obscure. Moreover, recent data suggest that MMR is frequently lost in many types of sporadic tumors (Han et al., 1993; Parsons et al., 1993; Risinger et al., 1994; Chong et al., 1994; Mironov et al., 1994; Orth et al., 1994; Shibata et al., 1994; Suzuki et al., 1994; Umar et al., 1994b; Wada et al., 1994; Wooster et al., 1994; Liu et al., 1995). This supports the notion that loss of MMR may play a general role in tumor evolution.
The best studied MMR system with respect to both genetics and biochemistry is the *mutSL* pathway of *Escherichia coli* (reviewed by Modrich, 1991). Central in this pathway is the protein encoded by the *muts* gene, which binds to base mispairs and to loops of up to four unpaired nucleotides. After binding to the heteroduplex, the MutS-DNA complex is bound by the *mutL* gene product, triggering excision of a tract of single-stranded DNA of up to several kilobases that contains the mismatched nucleotide(s). The repair process is completed by resynthesis of the excised DNA strand and ligation of the remaining nick. MMR in *E. coli* is responsible for maintaining genome integrity in at least two ways: (i) by recognizing and repairing mispaired and extrahelical nucleotides that occur by, respectively, misincorporation and slippage during replication and (ii) by recognizing mismatches occurring in recombination intermediates between homologous but diverged sequences, leading to abortion of recombination. Consequently, *E. coli* strains deficient for either *mutS* or *mutL* have a pleiotropic phenotype: an increased mutation rate, including destabilization of simple-sequence repeats (also called microsatellites: the 'Replication Error⁺', or RER⁺ phenotype) and promiscuity in inter- and intragenomic recombination (the recombinator phenotype, Rayssiguier et al., 1989; Petit et al., 1991).
In many respects the biochemistry of MMR systems in eukaryotes resembles that of the *mutSL* system (Holmes et al., 1990; Varlet et al., 1990; Thomas et al., 1991; Fang and Modrich, 1993). Homologs of both *mutS* and *mutL* were found in eukaryotes. Based on mismatch binding *in vitro* and on the mutator and recombinator phenotypes of *Saccharomyces cerevisiae* mutants, the protein encoded by the yeast *MSH2* gene (Reenan .and Kolodner, 1992a) seems to be the functional homolog of MutS (Reenan and Kolodner, 1992b; Miret et al, 1993; Alani et al, 1995). In higher eukaryotes (*Xenopus laevis*, mouse and human) homologs of the yeast *MSH2* gene have recently been identified, based on either sequence homology, direct sequencing of the major mismatch-binding protein or by positional cloning (Fishel et al., 1993; Leach et al., 1993; Palombo et al., 1994; Varlet et al., 1994). An apparent difference between MutS and MSH2, is the binding of the eukaryotic protein, when overproduced and purified from *E. coli* or yeast, to stretches of up to 14 extrahelical nucleotides (so called insertion-deletion-type loops, or IDLs) *in vitro* (Fishel et al., 1994b; Alani et al., 1995), suggestive of a role for Msh2 in the repair of these loops (Umar et al, 1994). Eukaryotic homologs of the *mutL* gene, called *MLH1, PMS1* and *PMS2* have been analyzed in yeast (Kramer et al., 1989a; Kramer et al., 1989b; Strand et al.. 1993; Prolla et al., 1994a; Prolla et al., 1994b) and human cells (Li and Modrich, 1995).
The precise role of these *mutS* and *mutL* homologs in MMR and the role of MMR in the maintenance of genome stability in mammalian cells remain to be established. In addition, the causative role of MMR in cellular sensitivity to simple methylating agents, as was suggested by the loss of MMR after selection of cells for tolerance to these agents (Branch et al., 1993; Kat et al., 1993) and by the methylation tolerance of a MMR-deficient rumor cell line (Koi et al., 1994) remains to be explored.
To investigate the involvement of Msh2 in mammalian MMR and the roles of MMR in maintaining genome stability we have generated mouse Embryonic Stem (ES) cell lines which lack both copies of the *Msh2* gene. We show that *Msh2*-deficient cells lack binding to some specific mismatches, have acquired a mutator phenotype and are tolerant to simple methylating agents. Moreover, these cells have lost the barrier to recombination between homologous but diverged sequences. Taken together, these results suggest that MMR is essential for the preservation of multiple aspects of genome stability in mammalian cells.
To study the effect of MMR deficiency on the etiology and evolution of cancer we generated mice lacking one or both copies of the *Msh2* gene in part or all of their cells. Although heterozygous mice, a model for HNPCC, remain healthy to at least 8 months of age, a significant fraction of both chimeric and fully homozygous *Msh2* knock-out mice succumbs to lymphomas. These results provide evidence that loss of MMR-controlled genome stability can accelerate the development of cancer.

### Targeted disruption of the Msh2 gene in mouse ES cells

Heterozygous ES cell lines s*Msh2*-42 and s*Msh2*-55, carrying a hygromycin-resistance gene inserted between codons 588 and 589 of one allele of the *Msh2* gene. were generated by gene targeting using the construct shown in Fig. 1A. Cell line d*Msh2*-9, carrying the *hyg* marker in both alleles of the *Msh2* gene, was obtained by selecting cell line *sMsh2*-55 for duplication of the chromosome carrying the targeted *Msh2* allele at an elevated hygromycin B concentration, a method that was previously shown to be effective using the neomycin-resistance marker (Mortensen et al, 1992). Targeting events were detected by Southern blot analysis, using probes flanking both sides of the targeting construct (Fig. 1A). An example of such a Southern blot, demonstrating the status of the *Msh2* alleles in cell lines wt-2 (a control cell line with the targeting construct integrated by non-homologous recombination), s*Msh2*-55 and d*Msh2*-9 is shown in Fig. 1B. Diploidy of cell lines s*Msh2*-55 and d*Msh2*-9 was verified by karyotyping (not shown).
The *hyg* marker was inserted, in the antisense orientation, N-terminally of the putative ATP binding site (Fig. 1A) in a region where (in the human gene) truncations were found in many HNPCC tumors (Liu et al., 1994). Moreover, no *Msh2* transcript could be detected in ES cell line d*Msh2*-9 by Northern blotting (not shown), indicating that the *hyg* marker strongly suppressed *Msh2* expression. The genotype of ES cell line d*Msh2*-9 will therefore be indicated by *Msh2*-/-.

### Mismatch binding in Msh2-/- cells

Two different mismatch-binding activities have been described in mammalian cell extracts using gel-retardation assays: (i) An activity that recognises G.T mismatches (Jiricny et al., 1988: Stephenson and Karran, 1989; Griffin and Karran, 1993), an extrahelical TG dinucleotide (Aquilina et al; 1994) representing an intermediate in replicational slippage of a microsatellite and, weakly, G.A, G.G, A.C and G.U mismatches (Stephenson and Karran, 1989: Hughes and Jiricny, 1992). (ii) In one cell line, an independent activity was detected that recognizes A.C. mismatches and also pyrimidine-pyrimidine mismatches (Stephenson and Karran, 1989). Based on protein sequencing, the purified G.T-binding activity was shown to contain the MSH2 protein (Palombo et al., 1994). To characterize the mismatch-binding properties of the *Msh2*-/- ES cell line, we have performed gel-retardation assays using cell extracts of wild-type ES cell line wt-2 and *Msh2*-deficient line d*Msh2*-9. Extracts were incubated with radiolabelled 38-mer double-stranded oligonucleotides containing either a mismatch, an extrahelical dinucleotide, or a 14-nucleotide insertion-deletion-type loop (IDL), followed by electrophoresis on a nondenaturing polyacrylamide gel. Results of these experiments as shown in Fig. 2 clearly demonstrate binding activity in wild-type cell extracts to a G.T mismatch and to an extrahelical TG dinucleotide. Binding to both oligonucleotides was entirely absent in the extract of the *Msh2*-/- ES cell line, demonstrating the involvement of the Msh2 protein in this binding activity. As shown before (Hughes and Jiricny. 1992). binding was abolished by the inclusion of ATP in the binding'reaction (not shown). We were unable to detect specific binding to G.A. G.G. or A.C mismatches under the conditions used in wild-type or mutant cell extracts. In addition, no Msh2-dependent binding to a 14-nucleotide IDL was observed (Fig. 2).

### Msh2-/- cells have a mutator phenotype.

The *Msh2*-/- cell line was indistinguishable from the wild-type cell line wt-2 and the parental heterozygous cell line *sMsh2-55* with respect to growth and plating efficiency (not shown). To address the role of Msh2 in MMR, we determined the stability of two microsatellite markers in *Msh2-I-* cell line d*Msh2*-9 and *Msh2*+*l*+ line wt-2. For this purpose, 24 subclones, were derived of each cell line, which had undergone approximately 20 cell divisions since its generation. Chromosomal DNA, isolated from each expanded subclone was subjected to the polymerase chain reaction (PCR) using two end-labelled primer pairs (*D7Mit17* and *D14Mit15*; Dietrich et al., 1994). Whereas none of the wt-2 sublines showed any alteration in microsatellite length, clear length alterations in many d*Msh2*-9 sublines were seen for both microsatellites (8 out of 24 for marker *D7Mit17* and 6 out of 24 for marker *D14Mir15*, Fig.3). This observation strongly suggests that the Msh2 protein is involved in the repair of slipped replication intermediates. The microsatellite-slippage rate was estimated to be 10⁻² to 10⁻³ per generation.
We subsequently investigated the effect of loss of the *Msh2* gene on the mutation frequency of a functional gene. To this purpose, 6x10⁶ cells of cell lines wt-2 and d*Msh2*-9 were plated in the presence of 6-thioguanine (6-TG) to select for cells that have lost activity of the X-linked *Hprt* gene by mutation. Whereas no 6-TG-resistant colonies were seen in the wt-2 culture, 188 resistant colonies were present in the d*Msh2*-9 culture. This result extends the mutator phenotype of *Msh2*-/- cells to functional genes.

*Msh*2-/- cells are tolerant to N-methyl-N'-nitro-N-nitrosoguanidine (MNNG) MMR was suggested to mediate hypersensitivity to agents that methylate G residues at the O⁶ position. It is believed that MMR recognises the O⁶-meG.T mispair that occurs after erroneous incorporation of a thymidine nucleotide opposite to O⁶-meG during replication (Griffin et al., 1994). This is supposedly followed by excision and repair synthesis, again incorporating thymidine opposite to O⁶-meG, triggering a new round of MMR. The net result of this cycling between excision and resynthesis will be the presence of single-stranded regions at the site of O⁶-meG residues which will lead to double-stranded gaps when replicated during S phase, resulting in cell death (for reviews, see Karran and Bignami, 1992; Karran and Bignami, 1994). It is predicted that loss of MMR will prevent this process. thus conferring tolerance to simple methylating agents. To test this hypothesis directly, survival of *Msh2-I-* cell line d*Msh2*-9, *Msh*+/- cell line s*Msh2*-21 and wild-type cell line wt-2 was determined after exposure to a range of concentrations of N-methyl-N'-nitro-N-nitrosoguanidine (MNNG). In the growth medium, *O*⁶-benzylguanine was included to competitively inhibit endogenous methyltransferase activity that might otherwise remove the methyl groups added by MNNG (Dolan et al., 1990). Fig. 4 shows that the LD₅₀ for MNNG was increased 20-fold in the *Msh2-I-* cell line compared to the heterozygous and wild-type cell lines, directly proving the involvement of Msh2 in determining cellular sensitivity to MNNG. Heterozygous cell line s*Msh*2-21 displayed no increased tolerance to MNNG.

### Loss of suppression of recombination between diverged sequences in Msh2-/- cells.

In *E*. *coli* (Shen and Huang, 198b), Drosophila (Nassif and Engels, 1993) and mammalian cells (Waldman and Liskay, 1988. Te Riele et al, 1992), the efficiency of recombination between homologous DNA stretches is highly dependent on their sequence identity. We have previously demonstrated that in a gene targeting assay, homologous recombination at the *Retinoblastoma (Rb)* locus in ES cells, derived from mouse strain 129, was 50-fold more efficient with a 129-derived targeting construct than with a construct derived from a non-isogenic (BALB/c) mouse strain (Te Riele et al, 1992; Table 1). This construct contained 0.6% base sequence divergence with respect to the isogenic 129 construct. To provide additional evidence that suppression of recombination was solely dependent on the polymorphisms between the endogenous locus and the targeting DNA. we performed the inverse experiment, i.e. targeting of a BALB/c-derived ES cell line with the 129- and BALB/c-derived constructs. This experiment yielded the inverse result, i.e. a higher targeting efficiency with the BALB/c targeting construct than with the non-isogenic 129 targeting construct (Table 1). To investigate whether MMR is responsible for this antirecombination effect, we have repeated the *Rb* targeting experiment in the (129-derived) ES cell line d*Msh2*-9. We found that in the *Msh2*-/- cell line homologous recombination at the *Rb* locus with the non-isogenic construct was as efficient as with the isogenic construct (Table 1). This experiment demonstrates that Msh2 is involved in preventing homologous recombination between diverged DNA sequences.

### Lack of mismatch binding in Msh2-/- cell extracts

Whereas wild-type cell extracts display efficient binding to both a G.T mismatch as well as to an extrahelical TG dinucleotide, extracts of the ES cell line d*Msh2*-9 lack this binding (Fig. 2). suggesting that the Msh2 protein is essential for binding to these heteroduplexes. We were unable to detect specific binding activity to other mismatches under the conditions used. This result is compatible with binding studies by others which demonstrate that binding to a G·T mismatch is stronger than to other mismatches (Stephenson and Karran, 1989; Hughes and Jiricny, 1992) whereas the independent A.C binding activity is apparently cell-type specific (Jiricny et al., 1988; Stephenson and Karran, 1989). The methylation-tolerant cell lines 'clone B' and RajiF12 were also found to lack binding to both a G.T mismatch (Branch et al., 1993; Griffin et al., 1994) and to an extrahelical TG dinucleocide (Aquilina et al., 1994). Binding to an A.C mismatch was unaffected (Branch et al., 1993). Therefore, these cell lines may have lost the *Msh2* gene. Purified human MSH2, overproduced in *E*. *coli*, and purified yeast MSH2 were shown to bind to G.T mismatches (Fishel et al., 1994a; Alani et al., 1995), but also strongly to IDLs of up to 14 nucleotides (Fishel et al., 1994b; Alani et al., 1995). Surprizingly, we could not detect Msh2-dependent binding to a 14 nucleotide IDL in ES cell extracts. We are unable to explain this apparent discrepancy. In contrast to the binding that we observed in ES cell extracts and that of the GTBP protein, the MSH2-containing G.T mismatch-binding protein fraction purified from HeLa cells (Hughes and Jiricny, 1992; Palombo et al., 1994), the binding capacity of the purified MSH2 proteins was not abolished by ATP (Fishel et al., 1994b; Alani et al., 1995). This suggests that the purified MSH2 proteins behave differently from the naturally produced protein. The finding that in *Msh2*-/- cells length shifts in microsatellites were never larger than 4 nucleotides (representing two CA dinucleotides; Fig. 3), suggests that binding activity of Msh2 to extrahelical loops longer than 4 nucleotides is not required to maintain microsatellite stability. We are currently initiating *in vitro* repair assays using wild-type and *Msh2*-/- cell extracts to further elucidate this point.

### Mutator phenotype of Msh2-deficient cells

Cell line d*Msh2*-9 had acquired microsatellite instability (the RER⁺ phenotype), indicating failure to repair slipped replication intermediates. The frequency of microsatellite instability was similar to that of some RER⁺ tumor cell lines (Parsons et al., 1993; Bhattacharyya et al., 1994). The mutator phenotype of these cells also affects functional genes as evidenced by the rate of Hprt deficiency in a d*Msh2*-9 culture which was increased from undetectable (i.e, lower than 1.6x10⁻⁷) for wild-type cells to 3x10⁻⁵. This frequency is in agreement with that found in some RER⁺ tumor cell lines (Bhattacharyya et al., 1994; Shibata et al., 1994; Eshleman et al., 1995). although cell lines that have been selected for MMR deficiency using a methylating agent tend to have somewhat lower mutation frequencies (Goldmacher et al., 1986; Branch et al., 1993; Kat et al., 1993; Aquilina et al., 1994). Growth rate and plating efficiency of *Msh2*-deficient, heterozygous and wild-type cells were indistinguishable, suggesting that the mutator phenotype does not interfere with viability of the cells. This is remarkable, since both were seriously affected in some cell lines that were selected for MMR deficiency using methylating agents (Goldmacher et al., 1986). This suggests that in the latter cells, additional events may have taken place that interfere with cell viability. Based on the mutator phenotype of d*Msh2*-9 cells and on the binding studies described above, we conclude that Msh2 is involved in, and essential for, postreplicational MMR.

### Sensitivity to simple methylating agents is mediated by MMR

Sensitivity of cells to agents that methylate DNA at the O⁶ position of G residues has been, attributed to futile attempts of MMR to correct the resulting O⁶-meG.T basepairs (see results). This hypothesis is supported by two observations: (i) Prolonged selection of cells for tolerance to Methylnitrosourea or MNNG frequently results in loss of MMR (Kat et al., 1993) and loss of G.T-binding activity (Branch et al., 1993; Griffin et al., 1994). (ii) An RER⁺ tumor cell line was found to be tolerant to these agents and sensitivity was restored by introduction of the chromosome containing a wild-type allele of the defective mismatch-repair gene (Koi et al., 1994). Our finding that *Msh2*-deficient cell line d*Msh2*-9 had acquired tolerance to MNNG provides direct evidence in support of this hypothesis. This finding may have important clinical implications. The triazene class of carcinostatic drugs probably derives its cytotoxic effect from methylation at the O⁶ position of G residues (Michejda et al., 1994). Whereas MMR-deficient tumors are supposedly tolerant to these drugs, they may elicit severe cytotoxity in normal tissues of the patient. In addition, the persistence of O⁶- meG.T mispairs in MMR-deficient tumor cells might lead to increased G to A transitions (Aquilina et al., 1993), resulting in acceleration of tumor progression. Finally, the finding that simple methylating agents select, or induce, MMR deficiency in cells has led to the hypothesis that this process is involved in determining the tisssue specificity of tumorigenesis in HNPCC patients, due to the presence of such agents in the intestine (Karran and Bignami, 1994; Varlet et al., 1994).

### MMR edits homologous recombination in mammalian cells

Previously, it has been shown that the presence of less than 1% nucleotide difference can strongly suppress homologous recombination in mammalian cells (Waldman and Liskay. 1988, Te Riele et al., 1992). Here. we have extended these results and we have demonstrated that *Msh2*-/- cells have lost this heterology-dependent suppression of recombination. Based on results obtained in *E*. *coli* (Worth et al., 1994), this function is probably mediated by the Msh2 protein. by recognizing mismatches that occur during heteroduplex formation between two not perfectly complementary DNA strands. This may directly destabilize the heteroduplex or, alternatively. prevent elongation of strand exchange. Similar results have recently been obtained with *MSH*2-deficient yeast strains (Alani et al., 1994; Selva et al., 1995), suggesting that this role of MMR is strongly preserved during evolution. In *E*. *coli*, loss of MMR has been shown to result in a high frequency of inter- and intrachromosomal rearrangements due to ectopic recombination between diverged sequences (Rayssiguier et al., 1989; Petit et al., 1991). More than the *E*. *coli* genome, mammalian genomes contain many repeated but diverged sequences. Examples of these are gene families. SINE sequences, LINE sequences and pseudogenes (Brosius and Gould, 1992; Nowak, 1994). Ectopic' recombination between these sequences would lead to genomic rearrangements that may either be detrimental for cell viability due to loss of essential genomic information or, alternatively, lead to activation of oncogehes or inactivation of tumor suppressor genes (Bouffter et al., 1993). Homologous recombination between identical DNA stretches should be allowed to proceed, however, to allow recombinational repair of double strand breaks using the (identical) sister chromatid as sequence donor. For this reason, mechanisms counteracting homologous, recombination when both recombining stretches contain divergencies, while permitting recombination to proceed when both stretches are identical, may be of great importance for the mammalian cell to enable repair without risking genome rearrangment. Based on the effect of *Msh*2-deficiency on gene targeting, it is likely that MMR plays this scrutinizing role.
Our finding that *Msh*2 acts not only anti-mutagenic but also anti-recombinogenic, suggests that MMR deficiency may contribute to tumorigenesis by elevating the rate of both point mutations and chromosomal rearrangements.

### Experimental procedures

### Disruption of Msh2 in mouse ES cells

Genomic *Msh2* fragments were obtained by screening a 129OLA-derived genomic DNA library with a murine *Msh2* cDNA probe (Varlet et al., 1994). The targeting construct was prepared by subcloning a 12.5 kbp *BamH* I fragment and inserting a hygromycin resistance gene (from PGK*hyg*, te Riele et al., 1990) into the unique *SnaB* I site located within an exon sequence of *Msh2*. Cloning procedures were performed according to Sambrook et al. ( 1989).
The targeting construct was seperated from vector sequences by gel electrophoresis, purified by electro elution and introduced into 129OLA-derived ES cell line E 14 by electroporation as described (te Riele et al., 1992). Electroporated cells were seeded onto gelatin-coated 10-cm dishes (10⁷ cells per plate) and subjected to hygromycin B selection (150 µg per ml) in BRL-conditioned medium (Hooper et al., 1987) the following day. After 10 days, individual hygromycin B-resistant colonies were randomly picked and expanded on mouse embryonic fibroblasts feeder layers. DNA was extracted from expanded colonies, digested with *Eco*R I and analyzed by Southern hybridization using probes flanking both sides of the targeting construct (Fig. 1A). Two cell lines were obtained out of 135 hygromycin B-resistant colonies showing bands diagnostic for correct integration of the *hyg* marker between codons 588 and 589 of one copy of the *Msh2* gene. These cell lines were designated s*Msh2*-42 and s*Msh2*-55. One cell line obtained from this experiment, designated wt-2, carrying a randomly integrated *hyg* gene, was used as an *Msh2*+/+ control.
To obtain an ES cell line carrying a disruption in both copies of *Msh2*, 10⁶ cells of cell line sMsh2-55 were plated onto 10-cm plates and cultured in BRL-conditioned medium containing 1.0 or 1.5 mg per ml of hygromycin B. After 12 days of of culturing in selective medium and 7 days in non-selective medium, 24 colonies were obtained which had survived 1.5 mg per ml of hygromycin B. DNA was extracted from expanded colonies, digested with *Eco*R I and analyzed by Southern hybridization. One cell line, designated d*Msh2*-9, carried two disrupted *Msh2* alleles, had lost the wild-type copy and contained the normal number of chromosomes. ES cell lines which had survived 1.0 mg per ml of hygromycin B still contained one disrupted and one wild-type *Msh2* copy. One of these *Msh2*+/- lines was designated s*Msh2*-21 and used for several experiments.

### Generation of Msh2 mutant mice

Chimeric mice were obtained by injecting 10-15 cells of ES cell lines s*Msh2*-55, s*Msh2*-42 and d*Msh2*-9 into C57B1/6 blastocysts. Male chimeras obtained with *Msh2*^{*+*}^{/}^{*-*} ES cells were crossed with wild-type 129OLA and FVB mice and found to transmit the mutated *Msh2* allele through the germ line. Homozygous *Msh2* mutant mice were obtained by intercrossing F1 heterozygotes.

### Gel shift assay

Preparation of cell extracts, annealing of oligonucleotides, binding of cell extracts to duplex oligonucleotides containing mismatched or extrahelical nucleotides, and nondenaturing polyacrylamide gelelectrophoresis were performed essentially as described (Stephenson and Karran, 1989). However, gelelectrophoresis was performed in TAE buffer rather than in TBE buffer. To obtain duplex oligonucleotides, the oligonucleotide **U**: 5'-GGGAAGCT-GCCAGGCCCCAGTGTCAGCCTCCTATGCTC-3' (sequences were derived from Aquilina et al., 1994) was radiolabelled and annealed with any of the following unlabelled oligonucleotides: **L-G.T**: 5'-GAGCATAGGAGGCTGACATTGGGGCCTGGCAGCTT-CCC-3' (resulting in a G.T mismatch); **L-G.A**: 5'-GAGCATAGGAGGCTGACAATGG-GGCCTGGCAGCTTCCC-3' (resulting in a G.A mismatch): **L-G.G**: 5'-GAGCATAGG-AGGCTGACAGTGGGGCCTGGCAGCTTCCC-3' (resulting in a G.G mismatch); **L-A.C**: 5'-GAGCATAGGAGGCTGACACCGGGGCCTGGCAGCTTCCC-3' (resulting in an A.C mismatch); **L-TG**:- 5'-GAGCATAGGAGGCTGACACTGTGGGGCCTGGCA-GCTTCCC-3' (resulting in an extrahelical TG dinucleotide): **L-HOM**: 5'-GAGCATAGGA-GGCTGACACTGGGGCCTGGCAGCTTCCC-3' (resultirig in a homoduplex); **L-LOOP14:** 5'-GAGCATAGGAGGCTGACACATACGTGAGTACTCTGGGGCCTGG-CAGCTTCCC-3' (resulting in an IDL loop of 14 extrahelical nucleotides). In all assays, a twofold excess of unlabelled homoduplex competitor oligonucleotide was included. As a positive control, a duplex oligonucleotide containing the binding site for the E2F family of transcription factors was used (Beijersbergen et al., 1995).

### PCR amplification of microsatellites

Subclones of ES cell lines d*Msh2*-9 and wt-2 were generated by seeding cells onto mouse embryonic fibroblasts feeder layers at a density of 10³ cells per 10 cm². At that time, the ES cell lines were in culture for approximately 20 divisions since their generation. Twenty-four colonies from each cell line were expanded. Chromosomal DNA.was isolated and subjected to the polymerase chain reaction using two end-labelled primer pairs (*D14Mit15* and *D7Mit17*, Dietrich et al., 1994). Amplified products were electrophoresed on a denaturing polyacrylamide gel.

### Mutation frequency

6x10⁶ cells of ES cell lines d*msh2*-9 and wt-2 were plated onto 486 cm² gelatin-coated tissue culture surface in BRL-conditioned medium. After two days, 6-Thioguanine was added at a concentration of 10 µg per ml. After two weeks the number of resistant colonies was counted.

### Sensitivity to MNNG

ES cell lines d*Msh*2-9, s*Msh*2-21 and wt-2 were seeded onto MEF feeder layers at a density of 10³ cells per 4 cm²· The following day, cells were exposed for one hour to MNNG ranging in concentration from zero to 36.45 µM in serum-free medium. After 4 days of incubation, cells were trypsinized, stained with Trypan blue and counted. During the whole procedure from one hour before exposure to MNNG, *O*⁶-benzylguanine (20 µM, kindly provided by A. Pegg) was included in the medium in order to competively inhibit endogenous methyltransferase activity that might otherwise remove the methyl groups added by MNNG (Dolan et al, 1990),

### Rb targeting with isogenic and non-isogenic DNA

Targeting and subsequent analysis of the *Rb* locus in a BALB/c-derived ES cell line (kindly provided by S. Rastan) and 129/OLA-derived ES cell lines d*Msh2*-9 were performed essentially as described (te Riele et al, 1992). The targeting constructs 129*Rb-neo* (derived from the 129OLA genome) and B/*cRb-neo* (derived from the BALB/c genome) carry an insertion of the pMC1*neo* marker in exon 19 of the *Rb* gene and differ approximately 0.6% at the nucleotide level (te Riele et al, 1992).

### References

Aaltonen. L.A., Peltomaki, P., Mecklin, J.-P., Jarvinen, H., Jass. J.R., Green, J.S., Lynch. H.T., Watson, P., Tallqvist, G., Juhola, M., Sistonen, P., Hamilton, S.R., Kinzler, K.W., Vogelstein, B., and de la Chapelle, A. (1994) Replication errors in benign and malignant tumors from hereditary nonpolyposis colorectal cancer patients. Cancer Res. *54*, 1645-1648.

Alani, E., Chi, N.-W., and Kolodner, R. (1995). The *Saccharomyces cerevisiae* Msh2 protein specifically binds to duplex oligonucleotides containing mismatched DNA base pairs and insertions. Genes Development *9*, 234-247.

Alani, E., Reenan, R. A. G., and Kolodner, R. D. (1994). Interaction beween mismatch repair and genetic recombination in *Saccharomyces cerevisiae*. Genetics *137*, 19-39.

Aquilina, G., Biondo, R., Doglotti, E., and Bigaami, M. (1993). Genetic consequences of tolerance to methylation DNA damage in mammalian cells. Carcinogenesis *14*, 2097-2103.

Aquilina, G., Hess, P., Branch, P., MacGeoch, C., Casciano, I., Karran, P., and Bignami, M. (1994). A mismatch recognition defect in colon carcinoma confers DNA microsatellite instability and a mutator phenotype. Proc. Natl. Acad. Sci. USA *91*, 8905-8909.

Beijersergen, R. L., Carlée, L., Kerkhoven, R. M., and Bernards, R. (1995). Regulation of the retinoblastoma protein-related p107 by G1 cyclin complexes. Genes Development *in press*.

Bhattacharyya, N. P., Skandalis, A., Ganesh, A., Groden, J., and Meuth, M. (1994). Mutator phenotypes in human colorectal carcinoma cell lines. Proc. Natl. Acad. Sci. USA. *91*, 6319-6323.

Bicknell, D. C., Rowan; A., and Bodmer, W. F. (1994). β₂M- microglobulin gene mutations: a study of established colorectal cell lines and fresh tumors. Proc. Natl. Acad. Sci. USA *91*, 4751-4755.

Bouffler, S., Silver, A., and Cox, G. (1993). The role of DNA repeats and associated structures in genomic instability and neoplasia. BioEssays *15*, 409-412.

Branch, P., Aquilina, G., Bignami, M., and Karran, P. (1993). Defective mismatch binding and a mutator phenotype in cells tolerant to DNA damage. Nature *362*, 652-654.

Bronner. C. E., Baker, S. M., Morrison, P. T., Warren, G., Smith, L. G., Lescoe. M. K., Kane, M., Earabino, C., Lipford, J., Lindblom, A., Tannergård. P., Bollag, R. J., Godwin, A. R., Ward, D. C., Nordenskjøld, M., Fishel. R., Kolodner, R., and Liskay, R. M. (1994). Mutation in the DNA mismatch repair gene homologue *hMLH1* is associated with hereditary non-polyposis colon cancer. Nature *368*, 258-261.

Brosius, J., and Gould, S. J. (1992). On "genornenclature": a comprehensive (and respectful) taxonomy for pseudogenes and other "junk DNA". Proc. Natl. Acad. Sci. USA *89*, 10706-10710.

Chong, J.-M., Fukayama, M., Hayashi. U., Takiawa, T., Koike. M., Konishi, M., Kiuchi-Yanoshita, R., and Miyaki, M. (1994). Microsatellite instability in the progression of gastric carcinoma. Cancer Research *54*, 4595-4597.

Cohen, P. R., Kohn, S. R., and Kuizrock, R. (1991). Association of sebaceous gland tumours and internal malignancy: the Muir-Torre syndrome. Am. J. Med. *90*, 606-613.

Dietrich, W.F., Miller, J.C., Steen, R.G., Merchant. M., Damron, D., Nahf. R., Gross, A., Joyce. D.C., Wessel, M., Dredge, R.D., Marquis, A., Stein, L.D., Goodman, N., Page, D.C., and Lander, E.C. (1994). A genetic map of the mouse with 4,006 simple sequence length polymorphisms. Nature Genetics *7*,220-240.

Dolan, M. E., Moschel, R., and Pegg, A. E. (1990). Depletion of mammalian *O*⁶-alkylguanine-DNA alkyltransferase activity by *O*⁶-benzylguanine provides a means to evaluate the role of this protein in protection against carcinogenic and therapeutic alkylating agents. Proc. Natl. Acad. Sci. USA *87*, 5368-5372.

Eshleman, J. R., Lang, E. Z., Bowerfind, G. K., Parsons. R., Vogelstein, B., Willson, J. K. V., Veigl, M. L., Sedwick, W. D., and Markowitz, S. D. (1995). Increased mutation rate at the *hprt* locus accompanies microsatelite instability in colon cancer. Oncogene *10*, 33-37.

Fang, W., and Modrich. P. (1993). Human strand-specific mismatch repair occurs by a bidirectional mechanism similar to that of the bacterial reaction. J. Biol. Chem. *268*, 11838-11844.

Fishel, R., Ewel, A., and Lescoe, M. K. (1994). Purified human MSH2 protein binds to DNA containing mismatched nucleotides. Cancer Res. *54*, 5539-5542.

Fishel, R., Ewel. A., Lee, S., Lescoe, M. K., and Griffith. J. ( 1994). Binding of mismatched microsatellite DNA sequences by the human MSH2 protein. Science *266*, 1403-1405.

Fishel, R., Lescoe, M. K., Rao, M. R. S., Copeland, N. G., Jenkins, N. A., Garber, J., Kane, M., and Kolodner. R. (1993). The human mutator gene homolog *MSH2* and its association with hereditary nonpolyposis colon cancer. Cell *75*, 1027-1038.

Goldmacher, V. S., Cuzick, Jr., R. A., and Thilly, W., G. (1986). Isolation and partial characterization of human cell mutants differing in sensitivity to killing and mutation by methylnitrosourea and N-methyl-N'-nitro-N-nitrosoguanidine. J. Biol. Chem. *261*, 12462-12471.

Griffin, S., and Karran, P. (1993). Incision at DNA G.T mispairs by extracts of mammalian cells occurs preferentially at cytosine methylation sites and is not targeted by a separate G.T binding reaction. Biochemistry *32*, 13032-13039.

Griffin, S., Branch, P., Xu. Y. -Z., and Karran. P. (1994). DNA mismatch binding and incision at modified guanine bases by extracts of mammalian cells: Implications for tolerance to DNA methylation damage. Biochemistry *33*, 4787-4793.

Han, H.-J., Yanagisawa, A., Kato, Y., Park, J.-G., Nakamura, Y. (1993). Genetic instability in pancreatic cancer and poorly differentiated type of gastric cancer. Cancer Research *53*, 5087-5089.

Hattwell, L. (1992) Defects in a cell cycle checkpoint may be responsible for the genomic instability of cancer cells. Cell *71*, 543-546.

Hemminki, A., Peltomäki, P, Mecklin, J.-P., Järvinen, H., Salovaara. R., Nyström-Lahti, M, de la Chapelle, A., and Aaltonen, L.A. (1994) Loss of the wild type *MLH1* gene is a feature of hereditary nonpolyposis colorectal cancer. Nature Genetics *8*, 405-410.

Holmes Jr., J., Clark, S., and Modrich, P. (1990) Strand-specific mismatch correction in nuclear extracts of human and *Drosophila melanogaster* cell lines. Proc. Natl. Acad. Sci. USA. *87*, 5837-5841.

Hooper, M., Hardy, K., Handyside, A., Hunter, S., and Monk, M. (1987). HPRT-deficient (Lesh-Nyhan) mouse embryos derived from germline colonization by cultured cells. Nature, *326*, 292-295.

Hughes, M. J., and Jiricny, J. (1992). The purification of a human mismatch-binding protein and association of its associated ATPase and helicase activities. J. Biol. Chem. *267*, 23876-23882.

Jass, J. R., Stewart, S. M., Stewart, J., and Lane, M.R. (1994). Hereditary non-polyposis colorectal cancer: morphologies, genes and mutations. Mutat Res. *310*, 125-133.

Jiricny, J. (1994). Colon cancer and DNA repair: have mismatches met their match? Trends in Genetics *10*, 164-168.

Jiricny, J., Hughes, M., Corman, N., and Rudkin, B. B. (1988). A human 200-kDa protein binds selectively to DNA fragments containing G.T mismatches. Proc. Natl. Acad. Sci. USA *85*, 8860-8864.

Karran, P., and Bignami, M. (1992). Self-destruction and tolerance in resistance of mammalian cells to alkylation damage. Nucl. Acids Res. *20*, 2933-2940.

Karran, P., and Bianami, M. (1994). DNA damage tolerance, mismatch repair and genome instability. BioEssays *16*, 833-839.

Kat, A., Thilly W. G. Fang, W.-H., Longley, M. J., Li, G. -M., and Modrich, P. (1993). An alkylation-tolerant, mutator human cell line is deficient in strand-specific mismatch repair. Proc. Natl. Acad. Sci. USA *90*, 6424-6428.

Koi, M., Umar. A., Chauhan, D. P., Cherian, S. P., Carethers. J. M., Kunkel, T. A., and Boland. C. R. (1994). Human chromosome 3 corrects mismatch repair defiency and microsatellite instability and reduces N-methyl-N'-nitro-N-nitrosoguanidine tolerance in colon tumor cells with homozygous *hMLHl* mutation. Cancer Research *54*, 4308-4314.

Kolodner, R. D., Hall, N. R., Lipford, J., Kane, M. F., Rao, M. R. S., Morrison, P., Wirth. L., Finan, P. J., Bum, J., Chapman, P., Earabino. C., Merchant, E., and Bishop, D. T. (1994). Structure of the human MSH2 locus and analysis of two Muir-Torre kindreds for msh2 mutations. Genomics *24*, 516-526.

Kolodner, R, D., Hall, N. R., Lipford. J., Kane, M. F., Morrison, P., Wirth. L., Finan, P. J., Burn, J., Chapman, P., Earabino, C., Merchant, E., and Bishop, D. T. (1995). Structure of the human *MLH1* locus and analysis of a large hereditary nonpolyposis colorectal carcinoma kindred for mlhl mutations. Cancer Res.*55*, 242-248.

Kramer, B., Kramer. W., Williamson, M., and Fogel, S. (1989). Heteroduplex DNA correction in *Saccharomyces cerevisiae* is mismatch specific and requires functional *PMS* genes. Mol. Cell. Biol. *9*, 4432-4440.

Kramer, W., Kramer, B., Williamson, M. S., and Fogel, S. (1989). Cloning and nucleotide sequence of DNA mismatch repair gene *PMS1* from *Saccharomyces cerevisiae*: homology of PMS1 with procaryotic MutL and HexB. J. Bacteriology *171*, 5339-5346.

Lazar, V., Grandjouan, S., Bognel, C., Couturier, D., Rougier, P., Bellet, D., and Bressac-de Paillerets, B. (1994). Accumulation of multiple mutations in tumour suppressor genes during colorectal tumorigenesis in HNPCC patients. Hum. Mol. Genet. *3*,2257-2260.

Leach, F. S., Nicolaides. N. C., Papadopoulos, N., Liu. B., len, J., Parsons, R., Peltomaka, P., Sistonen, P., Aaltonen, L. A., Nystrom-Lahti, M., Guan, X. -Y., Zhang, L., Meltzer, P. S., Yu, J. -W., Kao, F. -T., Chen, D. J., Cerosaletti, K. M., Fournier, R. E. K., Todd, S., Lewis, T., Leach. R. J., Naylor, S. L., Weissenbach, J., Mecklin, J. -P., Järvinen, H., Petersen, G. M., Hamilton, S. R., Green, J., Jass, J., Watson, P., Lynch, H. T., Trent, J. M., de la Chapelle, A., Kinzler, K. W., and Vogelstein, B. (1993). Mutations of a *mutS* homolog in hereditary nonpolyposis coiorectal cancer. Cell *75*. 1215-1225.

Li, G.-M., and Modrich, P. (1995) Restoration of mismatch repair to nuclear extracts of H6 colorectal tumor cells by a heterodimer of human MutL homologs. Proc. Natl. Acad. Sci. USA *92*, 1950-1954.

Liu, B., Nicolaides, N.C., Markowitz, S., Willson, J. K. V., Parsons, R. E., Jen, J., Papadopoulos, N.. Peltomäki, P., de la Chapelle, A., Hamilton, S. R., Kinzler, K. W., and Vogelstein. B. ( 1995). Mismatch repair gene defects in sporadic colorectal cancers with microsatellite instablity. Nature Genetics *9*, 48-55.

Liu, B., Parsons, R. E., Hamilton, S. R., Petersen, G. M., Lynch, H. T., Watson, P., Markowitz, S., Willson, J. K. V., Green, J., de la Chapelle. A., Kinzler, K. W., and Vogelstein. B. (1994). *hMSH2* mutations in hereditary nonpolyposis colorectal cancer kindreds. Cancer Research *54*, 4590-4594.

Loeb, L. A. (1994). Microsatellite instability: marker of a mutator phenotype in cancer. Cancer Research *54*, 5059-5063.

Lynch, H. T., Smyrk, T. C., Watson, P., Lanspa, S. J., Lynch. J. F., Cavalieri, R. J., and Bolland, C. R. (1993). Genetics, natural history, tumor spectrum, and pathology of hereditary nonpolyposis colorectal cancer: an updated review. Gastroenterology *104*, 1535-1549.

Michejda, C. J., Smith , Jr., R. H., and Kroeger Koepke, M. B. (1994). DNA alkylation by triazenes and related compounds. In DNA adducts: identification and biological significance, K. Hemminki, A. Dipple, D. E. G. Shuker, F. F. Kadlubar, D. Segerbäck, and H. Bartsch, eds. (IARC scientific publications and Oxford University press), pp. 323-337.

Miret, J. J., Milla, M. G., and Lahue, R. S. (1993). Characterization of a DNA mismatch-binding activity in yeast extracts. J. Biol. Chem. *268*, 3507-3513.

Mironov, N. M., Aguelon, M. A. -M., Potapova, G. I., Omori. Y., Gorbunov, O. V., Klimenkov, A. A., and Yamasaki, H. (1994). Alterations of (CA)ₙ repeats and tumor suppressor genes in human gastric cancer. Cancer Res. *54*, 41-44.

Modrich, P. (1991). Mechanisms and biological effects of mismatch repair. Annu. Rev. Genet. *25*, 229-253.

Modrich, P. (1994). Mismatch repair, genetic stability, and cancer. Science *266*, 1959-1960.

Mortensen, R. M., Conner, D. A., Chao, S., Geisterfer-Lowrance, A. A. T., and Seidman, J. G. (1992). Production of homozygous mutant ES cells with a single targeting construct. Mol. Cell. Biol. *12*, 2391-2395.

Nassif, N., and Engels, W. (1993). DNA homology requirements for mitotic gap repair in *Drosophila*. Proc. Natl. Acad. Sci. USA *90*, 1262-1266.

Nicolaides, N. C., Papadopoulos, N., Liu, B., Wei, Y. -F., Carter, K. -C., Ruben, S. M., Rosen, C. A., Haseltine, W. A., Fleischmann, R. D., Fraser, C. M., Adams M. D., Venter, J. C., Dunlop, M. G., Hamilton, S. R., Petersen, G. M., de la Chapelle. A.. Vogelstein, B., and Kinzler, K. W. (1994). Mutations of two *PMS* homologues in hereditary nonpolyposis colon cancer. Nature *371*, 75-80.

Nowak, R. (1994). Mining treasures from 'junk DNA'. Science *263*, 608-610.

Orth, K., Hung, J., Gazdar, A., Bowcack, A., Mathis, J. M., and Sambrook, J. (1994). Genetic instability in human ovarian cancer cell lines. Proc. Natl. Acad. Sci. USA. *91*, 9495-9499.

Palombo, F., Hughes, M., Jiricny, J.. Truong, O., and Hsuan, J. (1994). Mismatch repair and cancer. Nature *367*, 417.

Papadopoulos, N., Nickolaides, N. C., Wei, Y. -F., Ruben, S. M., Carter, K. C., Rosen, C. A.. Haseltine W. A., Fieischmann, R. D., Fraser, C. M., Adams, M. D., Venter, J. C., Hamilton, S. R., Petersen, G. M., Watson, P., Lynch, H. T., Peltomäki, P., Mecklin, J. -P., de la Chapelle, A., Kinzler, K. W., and Vogelstein, B. (1994). Mutation of a *mutL* homolog in hereditary colon cancer. Science *263*, 1625-1629.

Parsons, R., LI, G. -M., Longley, M., Modrich, P., Liu, B., Berk, T., Hamilton, S. R., Kinzler, K. W., and Vogelstein, B. (1995). Mismatch repair deficiency in phenotypically normal human cells. Science *268*, 738-740.

Parsons, R., Li. G. -M., Longley, M. J., Fang, W., Papadopoulos, M., Jen, J., de la Chapelle, A., Kinzler, K. W., Vogelstein, B., and Modrich, P. (1993). Hypermutability and mismatch repair deficiency in RER⁺ tumor cells. Cell *75*, 1227-1235.

Petit, M. A., Dimpfl, J., Radman, M., and Echols, H. (1991). Control of large chromosomal duplications in *Escherichia coli* by the mismatch repair system. Genetics *129*, 327-332.

Prolla, T. A., Christie, D. -M., and Liskay, R. M. (1994). Dual requirement in yeast DNA mismatch repair for *MLH1* and *PMS1*, two homologs of the bacterial *mutL* gene. Mol. Cell. Biol. *14*, 407-415.

Prolla, T., Pang, Q., Alani, E., Kolodner, R. D., and Liskay, R. M. (1994). MLH1, PMS 1, and MSH2 interactions during the initiation of DNA mismatch repair in yeast. Science *265*, 1091-1093.

Rayssiguier, C., Thaler, D. S. and Radman, M. (1989). The barrier to recombination between *Escherichia coli* and *Salmonella typhimurium* is disrupted in mismatch-repair mutants. Nature *342*, 396-401.

Reenan, R. A. G., and Kolodner, R. (1992a). Isolation and characterization of two *Saccharomyces cerevisiae* genes encoding homologs of the bacterial HexA and MutS mismatch repair proteins. Genetics *132*, 963-973.

Reenan. R. A. G., and Kolodner, R. (1992b). Characterization of insertion mutations in the *Saccharomyces cerevisiae MSH1* and *MSH2* genes: evidence for separate mitochondrial and nuclear functions. Genetics *132*, 975-985.

Risinger, J. I., Berchuck, A., Kohler, M. F., Watson, P., Lynch, H. T., and Boyd, J. (1993). Genetic instability of microsatellites in endometrial carcinoma. Cancer Res. *53*, 5100-5103.

Sambrook, J., Fritsch, E. F., and Maniatis,T. (1989). Molecular cloning, a laboratory manual, 2nd. edition. Cold Spring Harbor Laboratory Press.

Selva, E., New. L., Crouse, G., and Lahue, R. S. (1995). Mismatch correction acts as a barrier to homeologous recombination in *Saccharomyces cerevisiae*. Genetics *139*. 1175-1188.

Shen, P., and Huang, H. V. (1986). Homologous recombination in *Escherichia coli:* Dependence on substrate length and homology. Genetics *112*,441-457.

Shibata. D., Peinado, M. A., Ionov, Y., Malkhosyan, S., and Perucho, M. (1994). Genomic instability in repeated sequences is an early somatic event in colorectal tumorigenesis that persists after transformation. Nature Genetics *6*, 273-281.

Steghenson, C., and Karran, P. (1989). Selective binding to DNA base pair mismatches by proteins from human cells. J. Biol. Chem. *264*, 21177-21182.

Strand, M., Prolla, T. A., Liskay, R. M., and Petes, T. D. (1993). Destabilization of tracts of simple repetitive DNA in yeast by mutations affecting DNA mismatch repair. Nature *365*, 274-276.

Suzuki, H., Harpaz, N., Tannin. L., Yin. J., Bell, J. D., Hontanosas, M., Groisman, C. 'M.. Abraham, J. M., and Meltzer, S. J. (1994). Microsatellite instability in ulcerative colitis-associated colorectal dysplasias and cancers, Cancer Res. *54*, 4841-4844.

Te Riele, H., Robanus Maandag, E., and Bems. A. (1992). Highly efficient gene targeting in embryonic stem cells through homologous recombination with isogenic DNA constructs. Proc. Natl. Acad. Sci. USA *89*, 5128-5132.

Te Riele, H., Robanus Maandag, E., Clarke, A., Hooper, M., and Bems, A. (1990). Consecutive inactivation of both alleles of the *pim-l* proto-oncogene by homologous recombination in embryonic stem cells. Nature *348*, 649-651.

Thomas, D. C., Roberts. J. D., and Kunkel, T. A. (1991). Heteroduplex repair in extracts of human HeLa cells. J. Biol. Chem. *266*, 3744-3751.

Umar, A., Boyer, J. C., and Kunkel, T. A. (1994a). DNA loop repair by human cell extracts. Science *266*, 814-816.

Umar, A., Boyer, J. C., Thomas, D. C., Nguyen, D. C., Risinger. J. I., Boyd, J., Ionov, Y., Perucho. M., and Kunkel. T. A. (1994b). Defective mismatch repair in extracts of colorectal and endometrial cancer cell lines exhibiting microsatellite instability. J. Biol. Chem. *269*, 14367-14370.

Varlet, I., Radman. M., and Brooks, P. (1990). DNA mismatch repair in *Xenopus* egg extracts: Repair efficiency and DNA repair synthesis for all single base-pair mismatches. Proc. Natl. Acad. Sci. USA *87*, 7883-7887.

Varlet, I., Pallard, C., Radman, M., Moreau, J., and de Wind, N. (1994). Cloning and expression of the *Xenopus* and mouse *Msh2* DNA mismatch repair genes. Nucl. Acids. Res. *22*, 5723-5728.

Vogelstein, B., and Kinzler. K. (1993). The multistep nature of cancer. Trends in Bioch. Sci. *9*, 138-141.

Wada, C., Shionoya, S., Fujino, Y., Tokuhiro, H., Akahoshi, T., Uchida, T., and Ohtani, H. (1994). Genomic instability of microsatellite repeats and its association with the evolution of chronic myelogenous leukemia. Blood *83*, 3449-3456.

Waldman, A. S., and Liskay, R. M. (1988). Dependence of intrachromosomal recombination in mammalian cells on uninterrupted homology. Mol. Cell. Biol. *8*, 5350-5357.

Wooster, R., Cleton-Jansen, A. -M., Collins, N., Mangion, J., Cornelis, R. S., Cooper, C. S., Gusterson, B. A., Ponder, B. A. J., von Deimlino, A., Wiestier, O. D., Comelisse. C. J., Devilee, P., and Stratton, M. R. (1994). Instability of short tandem repeats (microsatellites) in human cancers. Nature Genetics *6*, 152-156.

Worth, Jr., L., Clark, S., Radman, M., and Modrich, P. (1994). Mismatch repair proteins MutS and MutL inhibit RecA-catalyzed strand transfer between diverged DNAs. Proc. Natl. Acad. Sci. USA *91*, 3238-3241.

Young, J., Leggett, B., Gustafson, C., Ward, M., Searle, J., Thomas, L., Buttenshaw, R., and Chevenix-Trench, G., (1993). Genomic instability occurs in colorectal carcinomas but not in adenomas. Human Mutation *2*. 351-353.

Fig. 1. Targeted disruption of the mouse *Msh2* gene.
A: Top: schematic diagram of the mouse Msh2 protein. ATP binding and HTH: putative ATPase and helix turn helix domains, respectively (Varlet et al, 1994). The position at which the protein is disrupted by insertion of the Hyg marker is indicated. Bottom: the genomic *Msh2* locus; indicated below is the targeting construct carrying the *Hyg* marker inserted into a unique *Sna*B I site within an exon sequence of *Msh2*. The positions of the external probes used for detection of targeting events are indicated.
B: The *Msh2* locus in *Msh2*+/+ cell line wt-2, *Msh2*+/- cell line s*Msh2*-55 and *Msh2*-/- cell line d*Msh2*-9. Genomic DNA of these cell lines was digested with *Eco*RI and analyzed by Southern hybridization using probe I (Fig. 1A). Arrowheads indicate the positions of the wild type (wt, 20 kbp), and disrupted (ko, 7.5 kbp) alleles.

Fig. 2. Mismatch-binding activity in wild-type and *Msh2*-deficient cells.
Binding activity in extracts of *Msh2*+/+ cell line wt-2 and *Msh2*-/- cell line d*msh2*-9 to double-stranded oligonucleotides containing a mismatch (**G.T. G.A, G.G**, or **A.C**), an extrahelical dinucleotide (**Extrahelical TG**), or an extrahelical 14-nucleotide loop (**LOOP 14**) and a **Homoduplex** was assessed using a gel retardation assay. Sequences of the oligonucleotides are given under Experimental Procedures. Arrowheads indicate the positions of mismatch-specific complexes. **E2F**: Binding to an oligonucleotide carrying an E2F site served as a positive control (Beijersbergen et al, 1995).

Fig. 3. Microsatellite stability in wild-type and *Msh2* -deficient cells. Genomic DNA of 24 subclones derived from *Msh2+*/*+* cell line wt-2 and *Msh2-l-* cell line d*msh2*-9 was amplified by PCR using primer sets *D7Mit17* and *D14Mit15*. Arrowheads indicate microsatellite alleles with a detectable length alteration.

Fig. 4. Tolerance of *Msh2*-deficient cells to the simple methylating agent MNNG.
ES cell lines wt-2 (*Msh2*+/+), s*Msh2*-55 (*Msh2*+/-) and d*Msh2*-9 (*Msh2*-/-) were exposed to increasing amounts of MNNG for one hour in the presence of *O*⁶-benzylguanine. After four days of incubation. cells were trypsinized and counted.

**Table 1.**

| Homologous recombination has lost dependence on sequence identity in *Msh2* deficient ES cells. | | | |
|---|---|---|---|
| ES cells | Homologous recombination vs total no. G418^{R} col. with | | lsogenic vs non-isogenic |
| | 129*Rb-neo* | B/c*Rb-neo* | |
| BALBIc (*Msh2*+/+) | 1/68 (7.5%) | 16/72 (22%) | 15 x |
| 129OLA (*Msh2*+/+) | 33/94 (35%) | 1/144 (0.7%) | 50 x |
| 129OLA (*Msh2*-/-) | 42/185 (23%) | 47/184 (26%) | 0.9 x |
| 129*Rb-neo* and B/c*Rb-neo* are *Rb* targeting constructs, prepared from the 129 and BALB/c strains of mice, respectively, with 0.6% sequence divergency. Targeting frequencies in 1290LA (*Msh2*+/+) ES cells are derived from te Riele et al., 1992. | | | |

## Claims

1. A method for modifying the genome of a non-human mammalian cell deficient for the DNA mismatch repair gene Msh2 in vitro by homologous recombination between a specific target locus in the genome and a homologous but diverged targeting DNA sequence introduced into the cell deficient for the DNA mismatch repair gene Msh2 and said targeting sequence has up to 30% base sequence differences with respect to the target locus in the region where recombination can take place or said targeting sequence differs from the target locus to such a level that homologous recombination is normally strongly impeded.

2. The method according to claim 1, wherein the cell's mismatch repair system gene Msh2 is inactivated by disruption of both copies of the Msh2 gene.

3. The method according to claim 1, wherein the cell's mismatch repair system gene Msh2 is inactivated by introduction of antisense RNA constructs, driven by an appropriate promoter, thus functionally inactivating the Msh2 gene.

4. The method according to claim 1, wherein the cell's mismatch repair system gene Msh2 is inactivated by introduction of modified antisense oligodeoxynucleotides, thus functionally inactivating the Msh2 gene.

5. The method according to claim 1, wherein the cell's mismatch repair system gene Msh2 is inactivated by expression of a dominant negatively acting version of a gene involved in DNA mismatch repair.

6. The method according to claim 1, wherein the cell's mismatch repair system gene Msh2 is inactivated transitorily by saturation of the mismatch repair system gene Msh2 by the introduction into the cell of DNA molecules carrying one or more mis- or unpaired bases.

7. The method according to claims 1-6, wherein the target cell is an embryonic stem cell or a related cell type derived from any non-human mammalian species, to be used to generate chimeric animals and chimeric animals that can transmit the mutated genome through the germ line.

8. The method according to claims 1-6, wherein the target cell is a cell line derived from any mammalian species and cultured in vitro.

9. The method according to claims 1-6, wherein the target cell is derived from any organ of any mammalian species.

10. The method according to claims 1-6, wherein the target cell is derived from outbred organisms implicating that they contain nonidentical copies of homologous chromosomes.

11. The method according to claim 1, wherein the targeting DNA at the region where homologous recombination can take place is derived from the same species as the target cell.

12. The method according to claim 11, wherein the targeting DNA is modified in vitro at specific sites thus deliberately introducing sequence divergence.

13. The method according to claim 1, wherein the targeting DNA at the region where homologous recombination can take place is derived from the same species as the target cell but is nonisogenic with the DNA of the target cell, the two sequence differing up to 5% at the nucleotide level.

14. The method according to claim 1, wherein the targeting DNA at the region where homologous recombination can take place is derived from another species as the target cell, the targeting sequence and the target locus comprising up to 30% sequence divergence in the region where homologous recombination can take place.

15. The method according to claim 14, wherein the targeting DNA is a chromosomal DNA fragment carried on a YAC or cosmid vector.

16. The method according to claim 1, wherein the targeting DNA is an double- or single-stranded oligonucleotide consisting of 10-100 bases or basepairs of which one or several bases or basepairs differ from the target locus.

17. A method to test inactivation of the cellular non-human mammalian mismatch repair system gene Msh2 using the method according to claims 2, 3, 4, 5 or 6 and involving comparison of the targeting efficiency of isogenic versus nonisogenic DNA targeting constructs.

18. A method to test inactivation of the cellular mismatch repair system gene Msh2 using the method according to claims 2, 3, 4, 5 or 6 and involving intrachromosomal recombination between homologous but diverged DNA sequences.

19. A method to test homologous recombination using the method according to claims 11, 12, 13, 14, 15 or 16 and using a non-human embryonic stem cell line or any other cell line in which the mismatch repair system is inactived by disruption of both copies of the Msh2 gene.

20. The method according to claims 8, 9 or 10 providing cells or cell lines from mismatch repair deficient mice carrying a disruption in both copies of the Msh2 gene.

## Patentansprüche

1. Verfahren zur Modifizierung des Genoms einer nicht-humanen Säugerzelle, welche hinsichtlich des DNA-Mismatch-Reparatur-Gens Msh2 defizient ist, in vitro durch homologe Rekombination zwischen einem spezifischen Zielort in dem Genom und einer homologen, aber divergierenden auf das Ziel gerichteten DNA-Sequenz, die in die Zelle mit dem defizienten DNA-Mismatch-Reparatur-Gen Msh2 eingeführt wurde, wobei die auf das Ziel gerichtete Sequenz bis zu 30 % Basensequenzunterschiede bezüglich des Zielorts in dem Bereich, an der die Rekombination stattfinden kann, aufweist, oder wobei sich die auf das Ziel gerichtete Sequenz von dem Zielort in einem solchen Ausmaß unterscheidet, dass homologe Rekombination normalerweise stark behindert wird.

2. Verfahren gemäß Anspruch 1, wobei das Mismatch-Reparatursystem-Gen Msh2 der Zelle durch Zerstörung beider Kopien des Msh2-Gens inaktiviert ist.

3. Verfahren gemäß Anspruch 1, wobei das Mismatch-Reparatursystem-Gen Msh2 der Zelle inaktiviert ist durch Einbringung von Antisense-RNA-Konstrukten, gesteuert von einem passenden Promotor, wodurch das Msh2-Gen funktionell inaktiviert wird.

4. Verfahren gemäß Anspruch 1, wobei das Mismatch-Reparatursystem-Gen Msh2 der Zelle durch Einbringung von modifizierten Antisense-Oligodesoxynucleotiden inaktiviert ist, wodurch das Msh2-Gen funktionell inaktiviert wird.

5. Verfahren gemäß Anspruch 1, wobei das Mismatch-Reparatursystem-Gen Msh2 der Zelle durch Expression einer dominanten negativ wirkenden Version eines Gens, weiches an der DNA-Mismatch-Rreparatur beteiligt ist, inaktiviert wird.

6. Verfahren gemäß Anspruch 1, wobei das Mismatch-Reparatursystem-Gen Msh2 der Zelle zeitweilig inaktiviert wird durch Sättigung des Mismatch-Reparatursystem-Gens Msh2 durch die Einbringung von DNA-Molekülen in die Zelle, welche eine oder mehrere fehl- oder ungepaarte Basen tragen.

7. Verfahren gemäß Ansprüchen 1 bis 6, wobei die Zielzelle eine embryonale Stammzelle oder ein verwandter Zelltyp ist, abgeleitet aus jedweder nicht-humanen Säugerspezies, welche zur Erzeugung chimärer Tiere und chimärer Tiere, welche das mutierte Genom über die Keimbahn weitergeben können, verwendet werden soll.

8. Verfahren gemäß Ansprüchen 1 bis 6, wobei die Zielzelle eine Zelllinie ist, welche aus jedweder Säugerspezies abgeleitet und in vitro kultiviert wird.

9. Verfahren gemäß Ansprüchen 1 bis 6, wobei die Zielzelle aus jedwedem Organ aus jedweder Säugerspezies abgeleitet ist.

10. Verfahren gemäß Ansprüchen 1 bis 6, wobei die Zielzelle aus Auszucht-Organismen abgeleitet ist, was impliziert, dass sie nicht-identische Kopien von homologen Chromosomen enthalten.

11. Verfahren gemäß Anspruch 1, wobei die Targeting-DNA an der Region, an welcher homologe Rekombination stattfinden kann, aus derselben Spezies wie die Zielzelle abstammt.

12. Verfahren gemäß Anspruch 11, wobei die Targeting-DNA in vitro an spezifischen Stellen modifiziert ist, wodurch Sequenzdivergenz willkürlich eingeführt werden kann.

13. Verfahren gemäß Anspruch 1, wobei die Targeting-DNA an der Region, wo homologe Rekombination stattfinden kann, aus derselben Spezies wie die Zielzelle abgeleitet ist aber nicht-isogen mit der DNA der Zielzelle ist, wobei sich die zwei Sequenzen um bis zu 5 % auf der Nucleotidebene unterscheiden.

14. Verfahren gemäß Anspruch 1, wobei die Targeting-DNA an der Region, an welcher homologe Rekombination stattfinden kann, aus einer anderen Spezies als die Zielzelle abgeleitet ist, wobei die Targeting-Sequenz und der Zielort bis zu 30 % Sequenzdivergenz in der Region, an der homologe Rekombination stattfinden kann, umfassen.

15. Verfahren gemäß Anspruch 14, wobei die Targeting-DNA ein chromosomales DNA-Fragment ist, welches auf einem YAC- oder Cosmid-Vektor getragen wird.

16. Verfahren gemäß Anspruch 1, wobei die Targeting-DNA ein doppeloder einzelsträngiges Oligonucleotid ist, bestehend aus 10-100 Basen oder Basenpaaren, von denen eine oder mehrere Basen oder Basenpaare sich vom Zielort unterscheiden.

17. Verfahren zum Testen der inaktivierung des nicht-humanen zellulären Säuger-Mismatch-Reparatursystem-Gens Msh2 unter Anwendung des Verfahrens gemäß der Ansprüche 2, 3, 4, 5 oder 6 und beinhaltend einen Vergleich der Effizienz zur Zielanbindung von isogenen gegenüber nichtisogenen auf das Ziel gerichteten DNA-Konstrukten.

18. Verfahren zum Testen der Inaktivierung des zellulären Mismatch-Reparatursystem-Gens Msh2 unter Anwendung des Verfahrens gemäß der Ansprüche 2, 3, 4, 5 oder 6 und beinhaltend intrachromosomale Rekombination zwischen homologen aber divergierenden DNA-Sequenzen.

19. Verfahren zum Testen der homologen Rekombination unter Anwendung des Verfahrens gemäß der Ansprüche 11, 12, 13, 14, 15 oder 16 und unter Verwendung einer nicht-humanen embryonalen Stammzelllinie oder jeglicher anderen Zelllinie, in welcher das Mismatch-Reparatursystem durch Disruption beider Kopien des Msh2-Gens inaktiviert ist.

20. Verfahren gemäß Ansprüchen 8, 9 oder 10, welches Zellen oder Zelllinien aus Mismatch-Rreparatur-defizienten Mäusen vorsieht, welche eine Disruption in beiden Kopien des Msh2-Gens tragen.

## Revendications

1. Procédé de modification du génome d'une cellule de mammifère non humain présentant une déficience du gène Msh2 de réparation des mésappariements de l'ADN, in vitro par recombinaison homogène entre un locus cible spécifique dans le génome et une séquence d'ADN de ciblage homologue mais divergente introduite dans la cellule présentant une déficience du gène Msh2 de réparation des mésappariements de l'ADN, selon lequel la séquence de ciblage présente jusqu'à 30 % de différences dans les séquences de bases par rapport au locus cible situé dans la région où une recombinaison peut avoir lieu, ou la séquence de ciblage diffère du locus cible à un degré tel que la recombinaison homologue est normalement fortement entravée.

2. Procédé selon la revendication 1, selon lequel le gène Msh2 du système de réparation des mésappariements de la cellule est inactivé par disruption des deux copies du gène Msh2.

3. Procédé selon la revendication 1, dans lequel le gène du système de réparation des mésappariements Msh2 de la cellule est inactivé par l'introduction d'hybrides d'ARN antisens, activés par un promoteur approprié, inactivant ainsi fonctionnellement le gène Msh2.

4. Procédé selon la revendication 1, selon lequel le gène Msh2 du système de réparation des mésappariements de la cellule est inactivé par l'introduction d'oligodésoxynucléotides antisens modifiés, inactivant ainsi fonctionnellement le gène Msh2.

5. Procédé selon la revendication 1, selon lequel le gène Msh2 du système de réparation des mésappariements de la cellule est inactivé par l'expression d'une version exerçant une dominance négative d'un gène participant à la réparation des mésappariements de l'ADN.

6. Procédé selon la revendication 1, selon lequel le gène Msh2 du système de réparation des mésappariements de la cellule est inactivé transitoirement par saturation du gène du système de réparation des mésappariements par l'introduction dans la cellule de molécules d'ADN portant une ou plusieurs bases incorrectement ou non appariées.

7. Procédé selon les revendications 1 à 6, selon lequel la cellule cible est une cellule souche embryonnaire ou une cellule de type apparenté dérivée d'une espèce quelconque de mammifère non humain, devant être utilisée pour élaborer des animaux chimériques et des animaux chimériques qui peuvent transmettre le génome muté à travers la ligne germinale.

8. Procédé selon les revendications 1 à 6, selon lequel la cellule cible est une lignée cellulaire dérivée d'une espèce quelconque de mammifère et cultivée in vitro.

9. Procédé selon les revendications 1 à 6, selon lequel la cellule cible est dérivée d'un organe quelconque d'une espèce quelconque de mammifère.

10. Procédé selon les revendications 1 à 6, selon lequel la cellule cible est dérivée d'organismes non consanguins impliquant qu'ils contiennent des copies non-identiques des chromosomes homologues.

11. Procédé selon la revendication 1, selon lequel l'ADN de ciblage au niveau de la région où une recombinaison homologue peut avoir lieu est dérivé de la même espèce que la cellule cible.

12. Procédé selon la revendication 11, selon lequel l'ADN de ciblage est modifié in vitro au niveau de sites spécifiques introduisant par conséquent délibérément une divergence dans les séquences.

13. Procédé selon la revendication 1, selon lequel l'ADN de ciblage au niveau de la région où une recombinaison homologue peut avoir lieu est dérivé de la même espèce que la cellule cible mais n'est pas isogénique avec l'ADN de la cellule cible, les deux séquences présentant jusqu'à 5 % de différences au niveau des nucléotides.

14. Procédé selon la revendication 1, selon lequel l'ADN de ciblage au niveau de la région où une recombinaison homologue peut avoir lieu est dérivé d'une autre espèce que la cellule cible, la séquence de ciblage et le locus cible présentant jusqu'à 30 % de divergence dans les séquences au niveau de la région où une recombinaison homologue peut avoir lieu.

15. Procédé selon la revendication 14, selon lequel l'ADN de ciblage est un fragment d'ADN chromosomique transporté par un vecteur constitué d'un chromosome artificiel de levure ou d'un cosmide.

16. Procédé selon la revendication 1, selon lequel l'ADN de ciblage est un oligonucléotide à double ou simple brin composé de 10 à 100 bases ou paires de bases parmi lesquelles une ou plusieurs bases ou paires de bases diffèrent du locus cible.

17. Procédé pour tester l'inactivation du gène Msh2 du système de réparation des mésappariements d'une cellule de mammifère non humain utilisant le procédé selon l'une quelconque des revendications 2, 3, 4, 5 ou 6 et comportant la comparaison de l'efficacité de ciblage des hybrides d'ADN de ciblage isogéniques par rapport aux hybrides non isogéniques.

18. Procédé pour tester l'inactivation du gène Msh2 du système de réparation des mésappariements d'une cellule de mammifère utilisant le procédé selon l'une quelconque des revendications 2, 3, 4, 5 ou 6, et comportant une recombinaison intrachromosomique entre des séquences d'ADN homologues mais divergentes.

19. Procédé pour tester une recombinaison homologue utilisant le procédé selon l'une quelconque des revendications 11, 12, 13, 14, 15 ou 16, et utilisant une lignée de cellules souches embryonnaires non humaines ou une autre lignée cellulaire quelconque dans laquelle le système de réparation des mésappariements est inactivé par la disruption des deux copies du gène Msh2.

20. Procédé selon l'une quelconque des revendications 8, 9 ou 10, fournissant des cellules ou des lignées cellulaires provenant de souris présentant une déficience dans le système de réparation des mésappariements portant une disruption des deux copies du gène Msh2.
